# EUROPEAN PATENT APPLICATION

(11) **EP 1 731 608 A1**
(43) Date of publication of application: **13.12.2006**
(21) Application number: 05727836.8
(22) Date of filing: 29.03.2005
(51) Int. Cl.: C12N 15/11, A61K 45/00, A61P 19/02, A61P 29/00, A61P 43/00, C07K 14/47, C07K 14/705, C12N 1/15, C12N 1/19, C12N 1/21, C12N 5/10, C12P 21/02, C12Q 1/68, G01N 33/15

(54) **MARKER GENE FOR ARTHRORHEUMATISM TEST**

(30) Priority: 29.03.2004 JP 2004096989
(71) Applicant: Tokai University, Tokyo 151-0063 (JP); Inoko, Hidetoshi, Yokohama-shi, Kanagawa-ken 245-0002 (JP)
(72) Inventor: INOKO, Hidetoshi, Yokohama-shi, Kanagawa 2450002 (JP); TAMIYA, Gen, Tokushima 770-0005 (JP); GOJOBORI, Takashi, 4110026 (JP)
(74) Representative: Curtis, Philip Anthony
(86) International application number: PCT/JP2005/005904
(87) International publication number: WO 2005/093066

(57) **Abstract**

It is intended to identify rheumatoid arthritis susceptibility genes by a highly efficient, low-cost mapping method using microsatellites. In the present invention, novel rheumatoid arthritis susceptibility genes, that is, TNXB, NOTCH4, RAB6A, MPRL48, UCP2, and UCP3 genes, in the human genomic DNA sequence were identified by conducting case-control association analysis on rheumatoid arthritis by use of microsatellite polymorphic markers assigned at approximately 100-kb intervals to narrow down candidate regions and then conducting association analysis and linkage analysis with SNP as a marker.

## Description

### Technical Field

The present invention relates to rheumatoid arthritis susceptibility genes identified de novo by a gene mapping method using microsatellite polymorphic markers, and to use thereof.

### Background Art

Arthrorheumatism (Rheumatoid arthritis: RA) is a chronic inflammatory disease characterized by autoimmunity. RA, which exhibits progressive inflammation with meningeal cell overproliferation in joints, is pathologically classified into joint tissue diseases. The morbidity of RA with respect to population is high and reaches approximately 1% of various races. The familial aggregation and monozygotic twin concordance rates of RA have previously been reported to be relatively high, suggesting the presence of an inheriting factor in its pathogenesis. Indeed, it is known that in the family of a proband with RA, a closer relative of the proband has higher risk of recurrence. According to previous reports, the ratio of risk of the disease in the siblings (λs) of the proband falls within 2 to 10.

Among RA susceptibility genes previously found, the HLA-DRB1 locus in the HLA class III region on 6p21.3 has been thought to most strongly contribute to RA and estimated to account for 30 to 50% of total genetic risk. On the contrary, this also suggests the presence of other genes undiscovered having genetic contribution as strong as HLA-DRB1. Some of such other genes have been considered to reside in the HLA region and have linkage with HLA-DRB1. Many researchers have continuously conducted studies to identify those other genes by various approaches including genomewide linkage analysis such as sib-pair analysis (Non-Patent Documents 1 to 3) and genetic association analysis such as case-control analysis (case-control study) on candidate genes or chromosome regions (Non-Patent Documents 4 to 6). However, these studies fell short of the identification of all RA susceptibility genes and the full explanation of mechanisms of its onset.

An approach that examines the association between bases exhibiting single nucleotide polymorphisms (SNPs) in the human genomic DNA sequence and disease has received attention as a method for identifying novel disease-related genes or the like. However, SNPs are derived from one-nucleotide substitution on the genome and therefore result in only two alleles in general. In this approach, since only some SNPs, which are present within approximately 5 kb from a disease-related gene to be mapped, exhibit association, genome mapping with SNPs as polymorphic markers requires assigning an enormous number of SNPs as markers for analysis. Under the present circumstances, this approach is therefore applied only to a limited region narrowed down to some extent. On the other hand, a microsatellite polymorphic marker has many alleles and is characterized in that it exhibits association even at some position distant from a gene to be mapped. However, the microsatellite polymorphic marker presented problems in that too many polymorphic markers assigned make analysis difficult in light of time and labors, as with SNPs, while too few polymorphic markers assigned make marker spacings too large and might overlook a disease-related gene.

The present inventors have developed a gene mapping method using microsatellite polymorphic markers assigned at approximately 50-kb to 150-kb intervals on average and have found that a region where a disease-related gene or gene relating to human phenotypes with genetic factors is present can be identified at high efficiency and low cost by using the method (Patent Document 1).
Non-Patent Document 1: Conelis, F. et al., Proc. Natl. Acad. Sci. USA, 95, 10746 (1998)
Non-Patent Document 2: Shiozawa, S. et al., Int. Immunol., 10, 1891 (1998)
Non-Patent Document 3: Jawaheer, D. et al., Am. J. Him. Genet., 68, 927 (2001)
Non-Patent Document 4: Okamoto, K., et al., Am. J. Hum. Genet., 72, 303 (2003)
Non-Patent Document 5: Suzuki, A. et al., Nat. Genet., 34, 395 (2003)
Non-Patent Document 6: Tokuhiro, S. et al., Nat. Genet., 35, 341 (2003)
Patent Document 1: International Publication of WO01/79482

### Disclosure of the Invention

Accordingly, an object of the present invention is to identify novel RA susceptibility genes by applying a precise mapping method with microsatellite markers capable of completely identifying disease susceptibility genes at higher cost efficiency than that of conventional approaches of SNP association analysis to multifactorial disorder RA for the first time. A further object of the present invention is to eventually develop the effective prevention/treatment of RA by collecting data on RA pathogenesis or onset mechanisms on the basis of the information of the identified RA susceptibility genes or RA-related proteins as expression products of the genes and performing proper screening.

In the present invention, a gene mapping method using microsatellite (hereinafter, referred to as "MS") was used to identify novel RA susceptibility genes whose associations with RA had not been known so far.

The RA susceptibility genes identified de novo by the present invention are TNXB and NOTCH4 genes (chromosome 6) as well as RAB6A, MPRL48, FLJ11848, UCP2, and UCP3 genes (chromosome 11) in the human genomic DNA sequence. The present inventors conducted the association analysis of RA with SNPs present in the genomic DNA sequences of these de novo-identified genes, and found statistically significant association for the first time.

Thus, in the first aspect, the present invention provides a marker gene for arthrorheumatism test consisting of a consecutive partial DNA sequence comprising at least one base exhibiting single nucleotide polymorphism present in a TNXB, NOTCH4, RAB6A, MPRL48, UCP2 or UCP3 gene in the human genomic DNA sequence, or of a complementary strand of the partial DNA sequence.

In the second aspect, the present invention provides a test method and test kit for RA using the marker gene.

### Brief Description of the Drawings

Figure 1 is a diagram showing the positions where MS markers used in Example of the present application are mapped on chromosomes;
Figure 2 is a diagram showing the mapping and P-values of MS markers used in a first-phase screening. The P-values of 133 MS markers exhibiting significance are indicated by circles (o) ;
Figure 3 is a diagram showing the positions where MS and SNP markers selected in Example of the present application are mapped on chromosomes, blocks predicted by EM and Clark algorithms, and P-values for allele frequency;

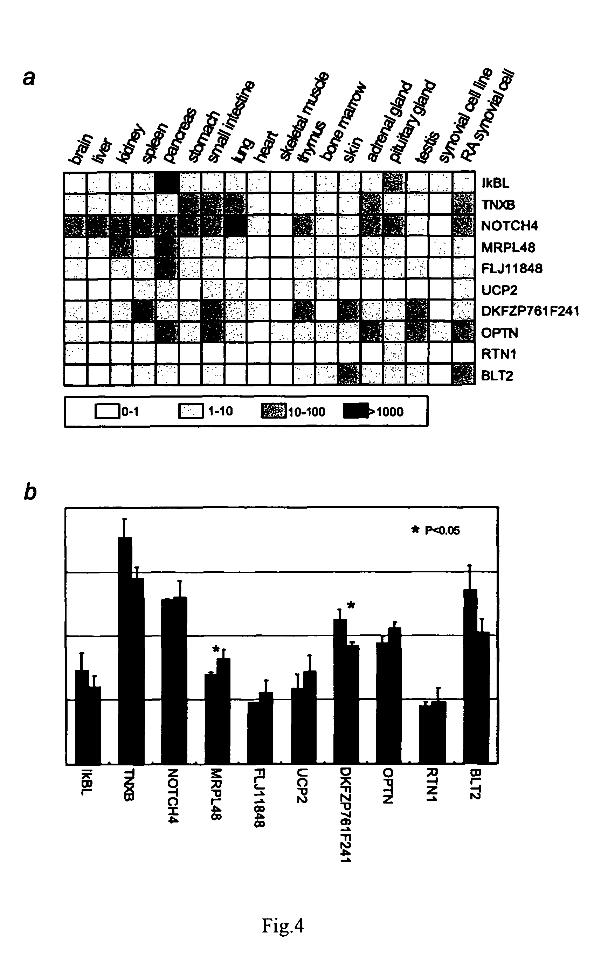

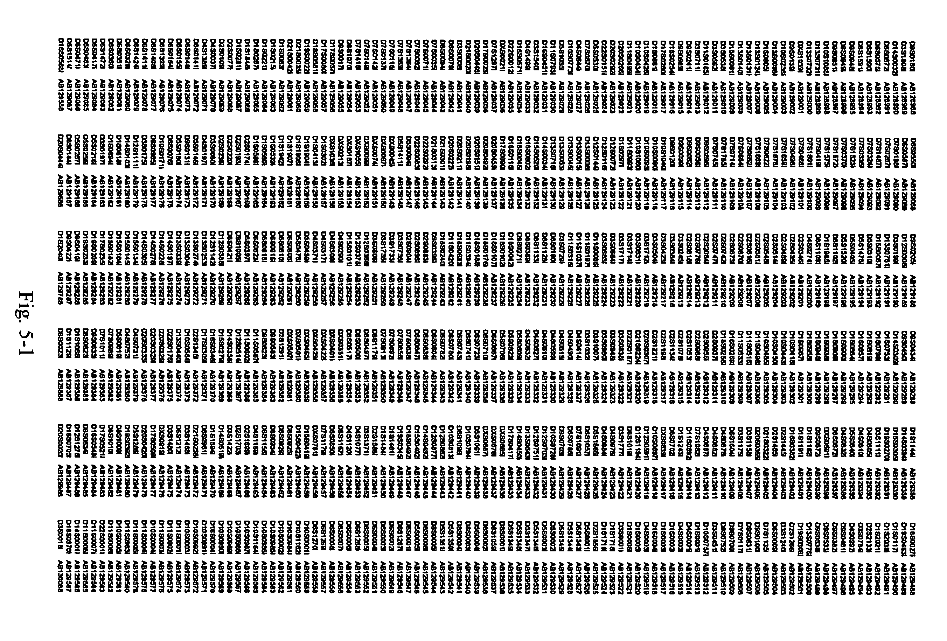

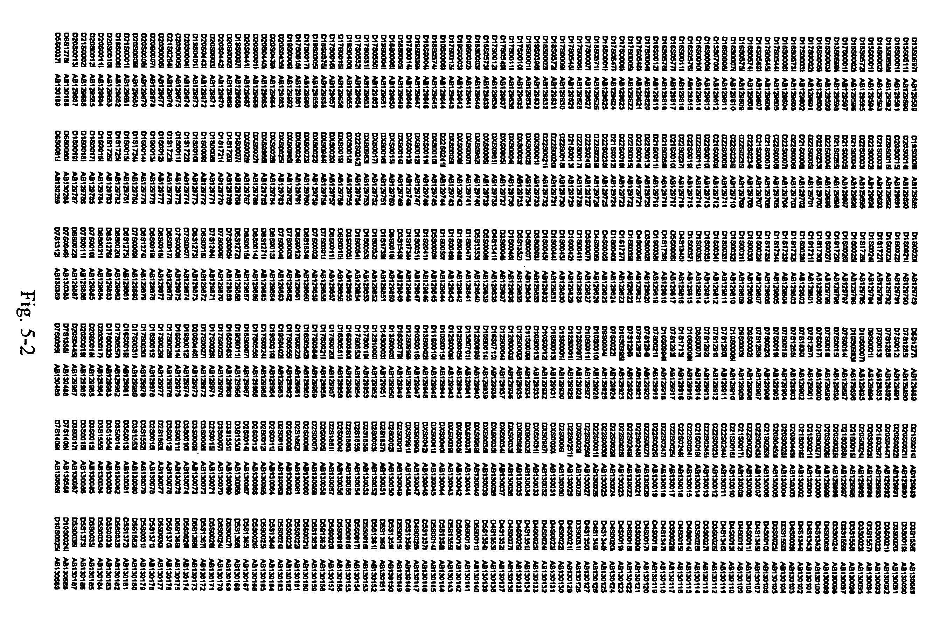

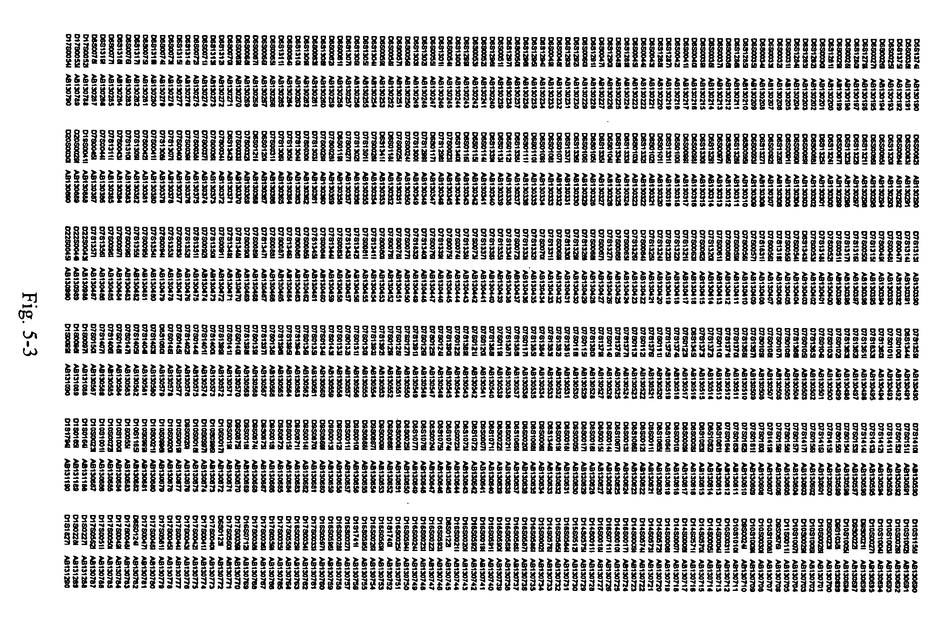

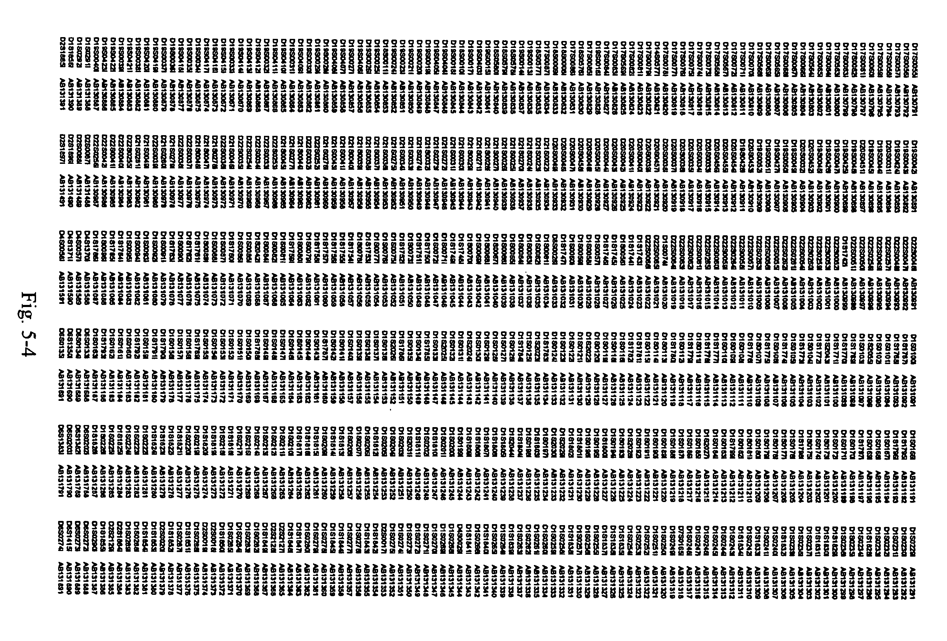

Figure 4 is a diagram showing the distribution of tissue expression of RA susceptibility genes identified by the present invention, and so on;
Figure 5-1 is a list showing information on the designations (left in each column) and Genbank registration numbers (right in each column) of microsatellite markers and primers used in the present invention;
Figure 5-2 is a list showing information on the designations (left in each column) and Genbank registration numbers (right in each column) of microsatellite markers and primers used in the present invention;
Figure 5-3 is a list showing information on the designations (left in each column) and Genbank registration numbers (right in each column) of microsatellite markers and primers used in the present invention;
Figure 5-4 is a list showing information on the designations (left in each column) and Genbank registration numbers (right in each column) of microsatellite markers and primers used in the present invention;
Figure 5-5 is a list showing information on the designations (left in each column) and Genbank registration numbers (right in each column) of microsatellite markers and primers used in the present invention;

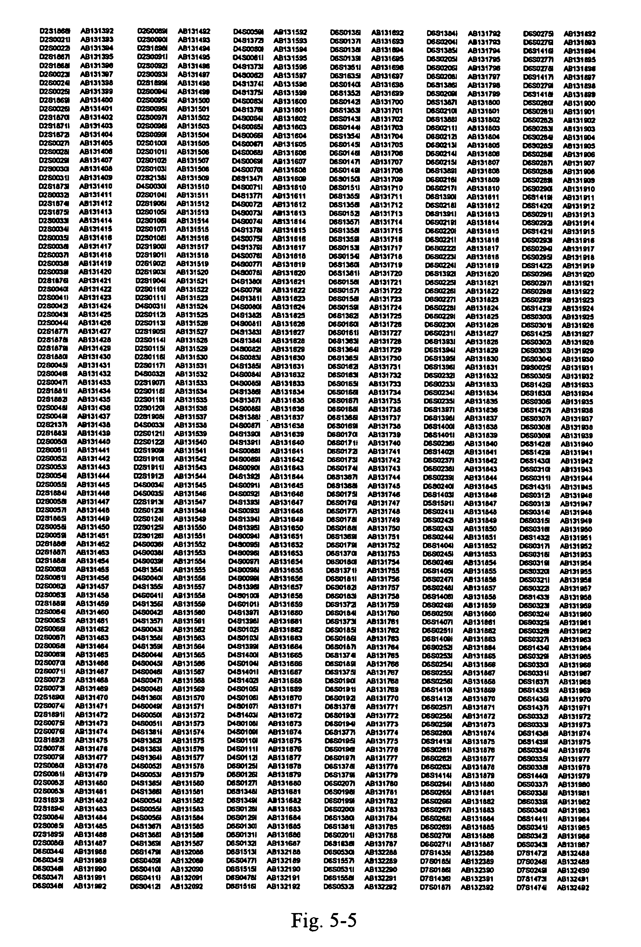

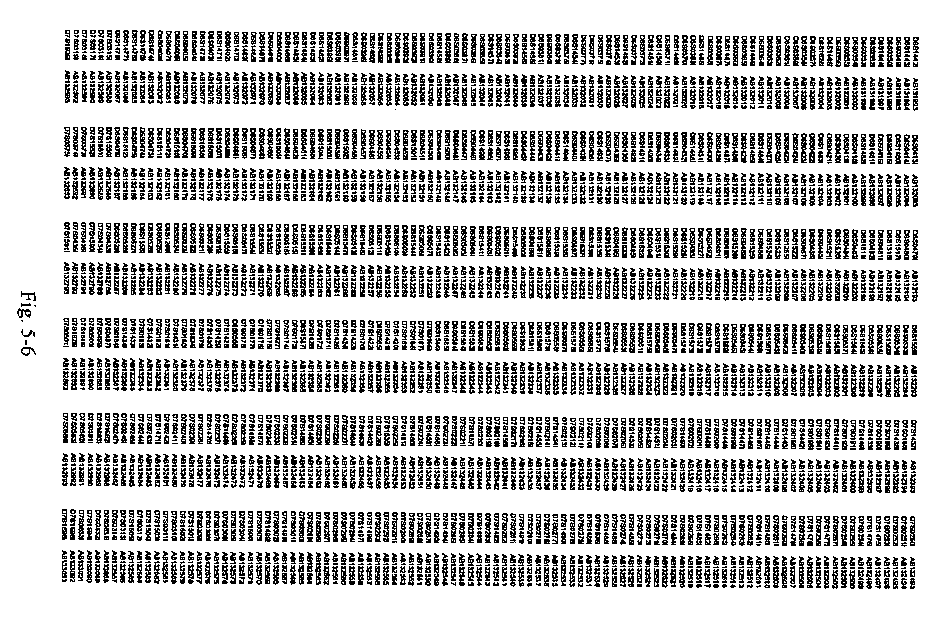

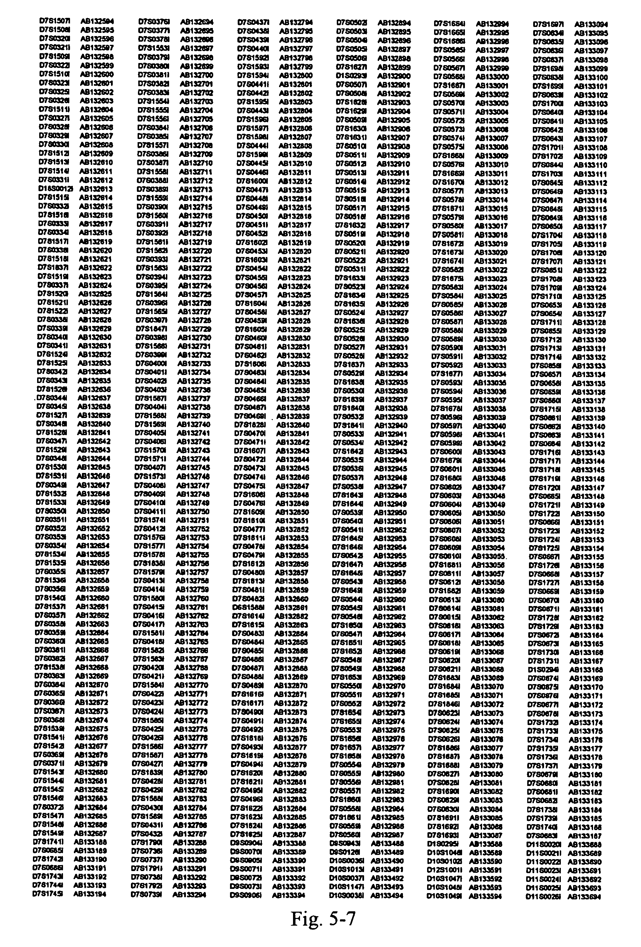

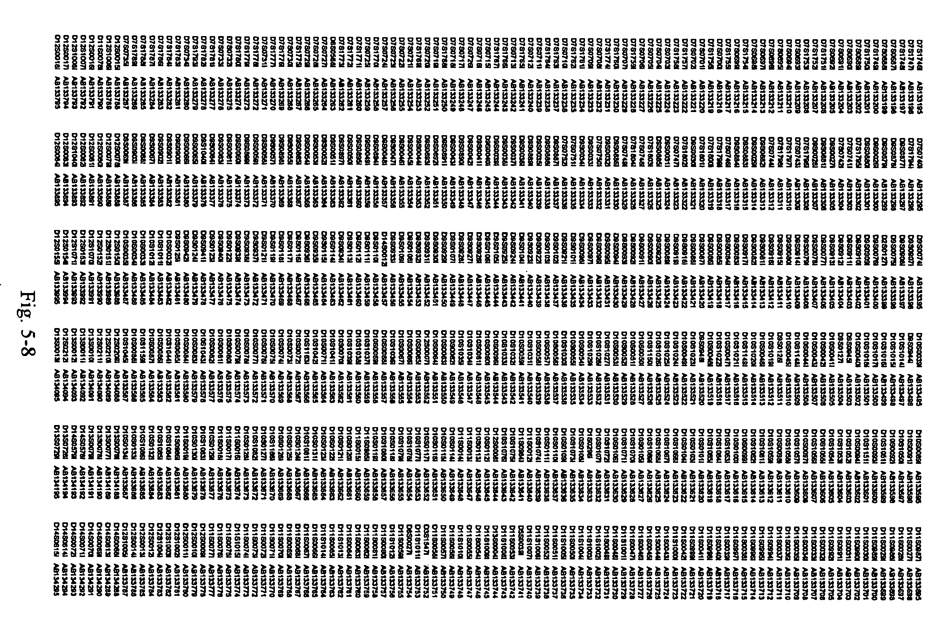

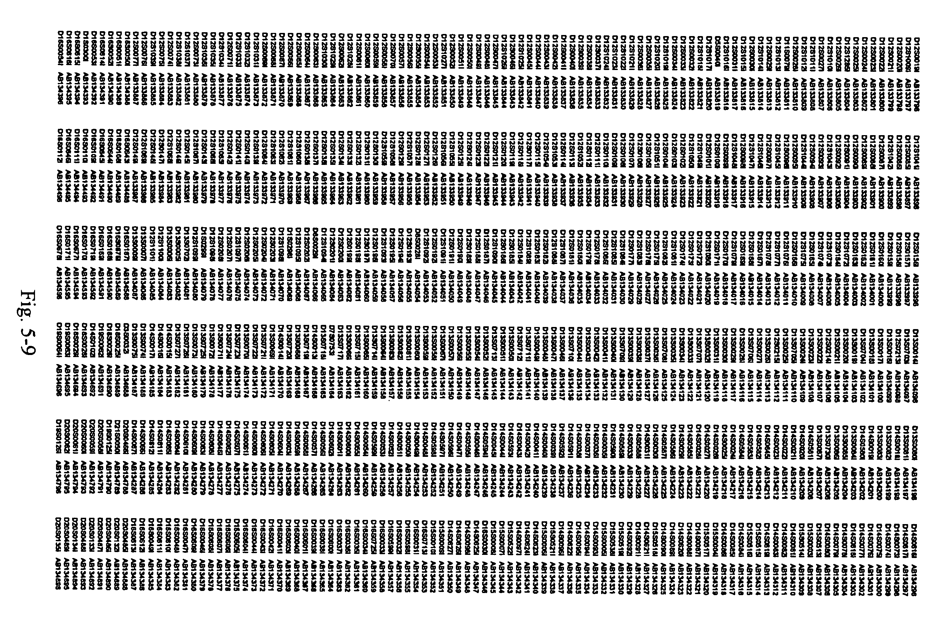

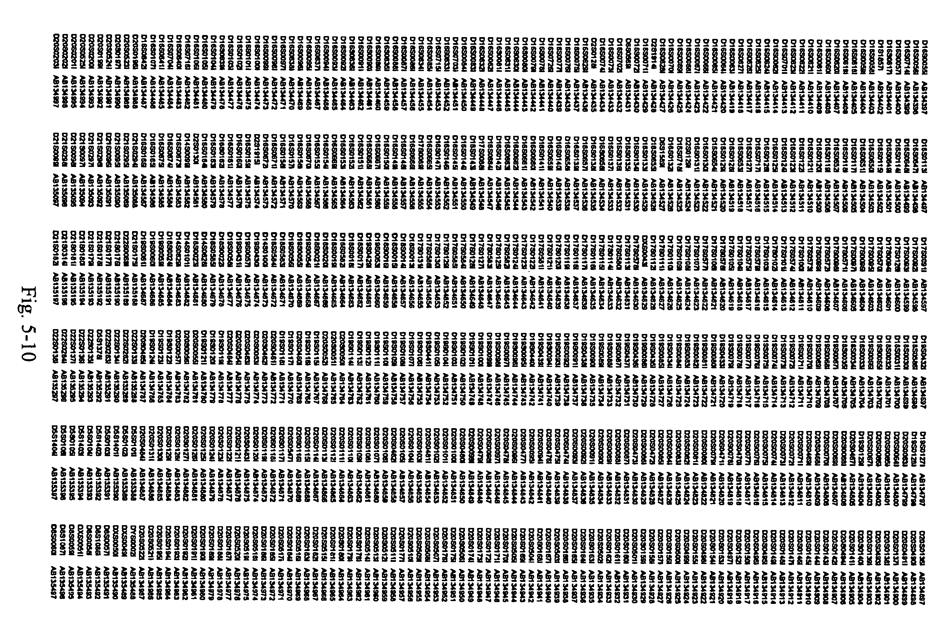

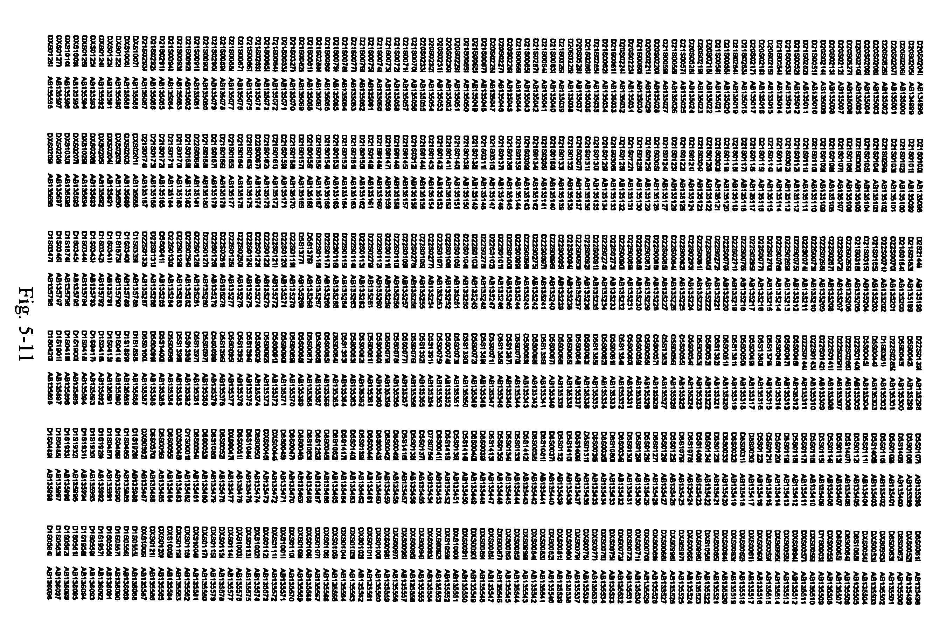

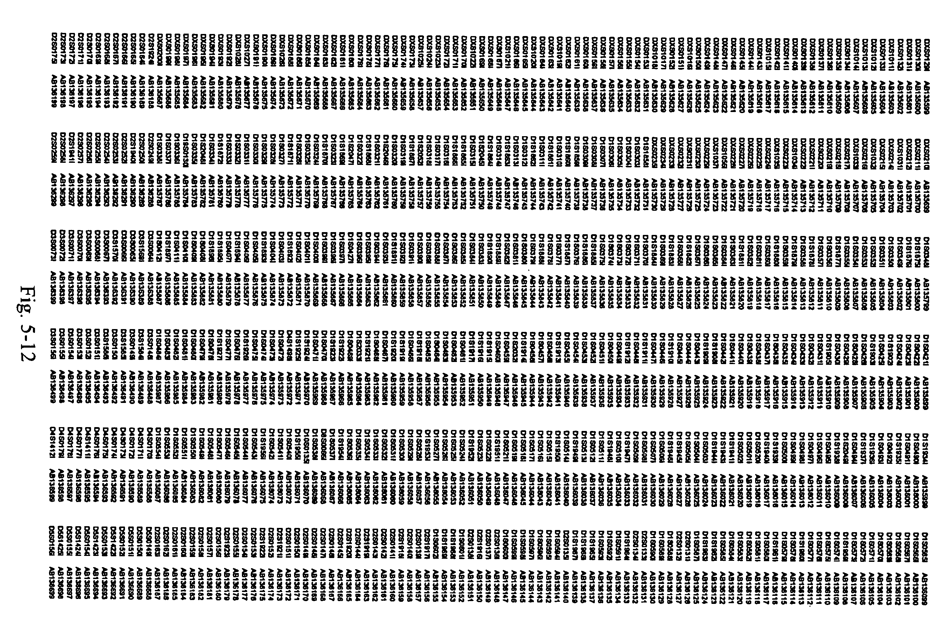

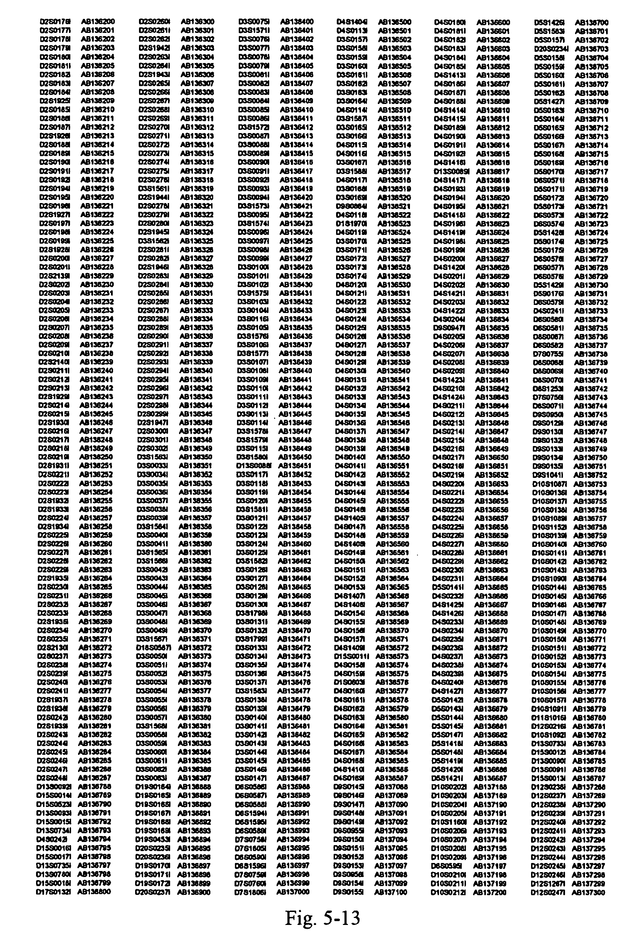

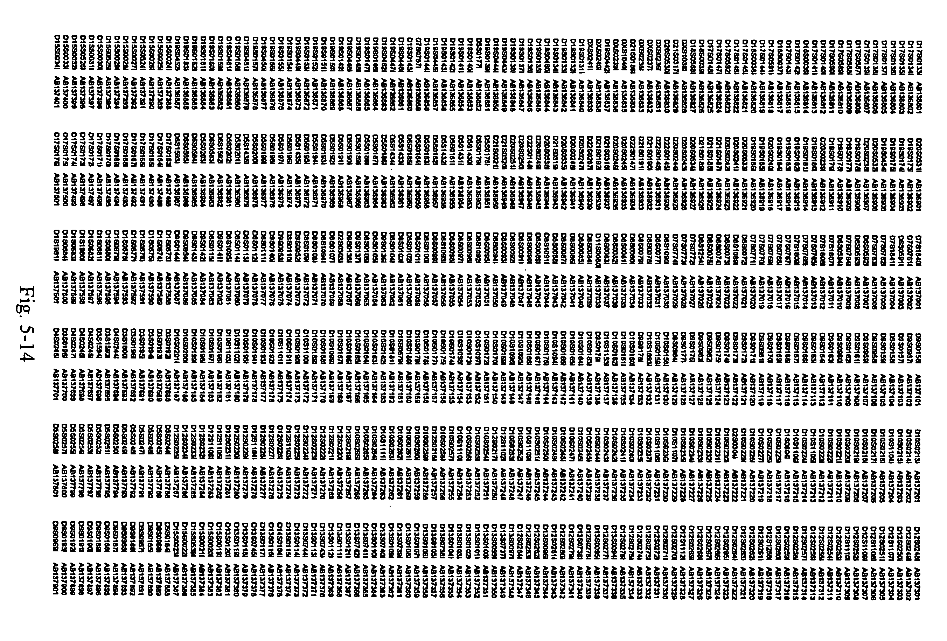

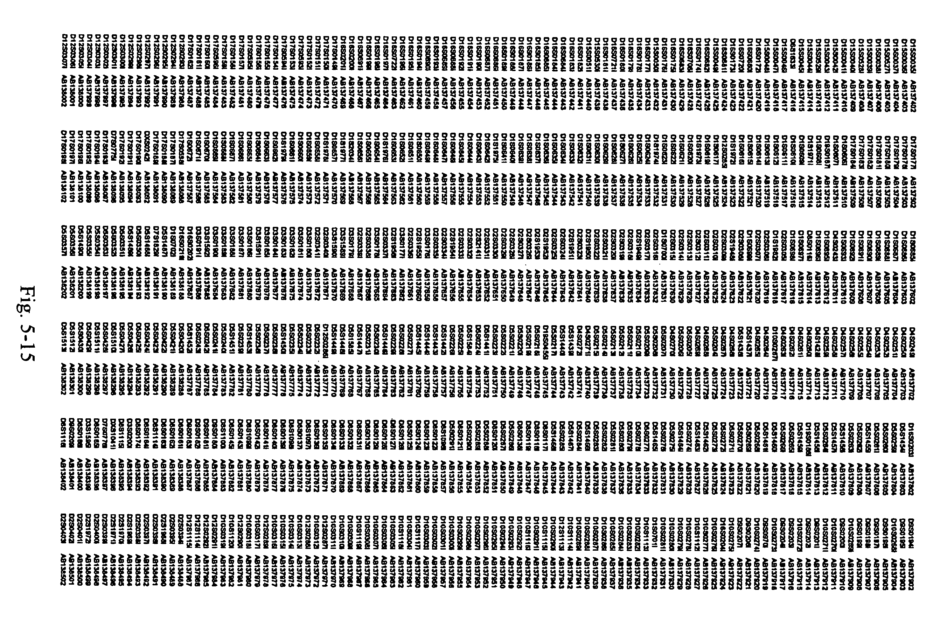

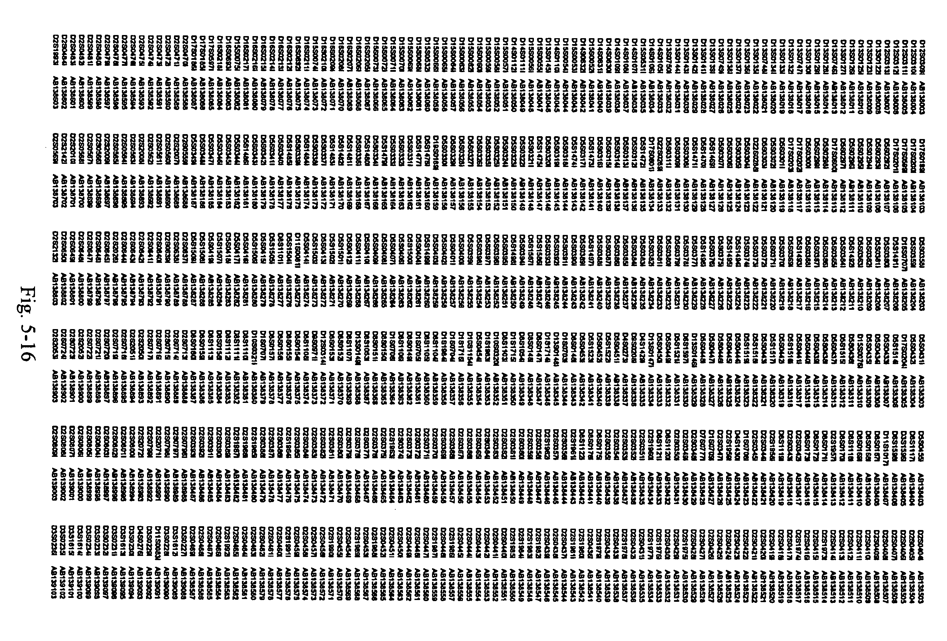

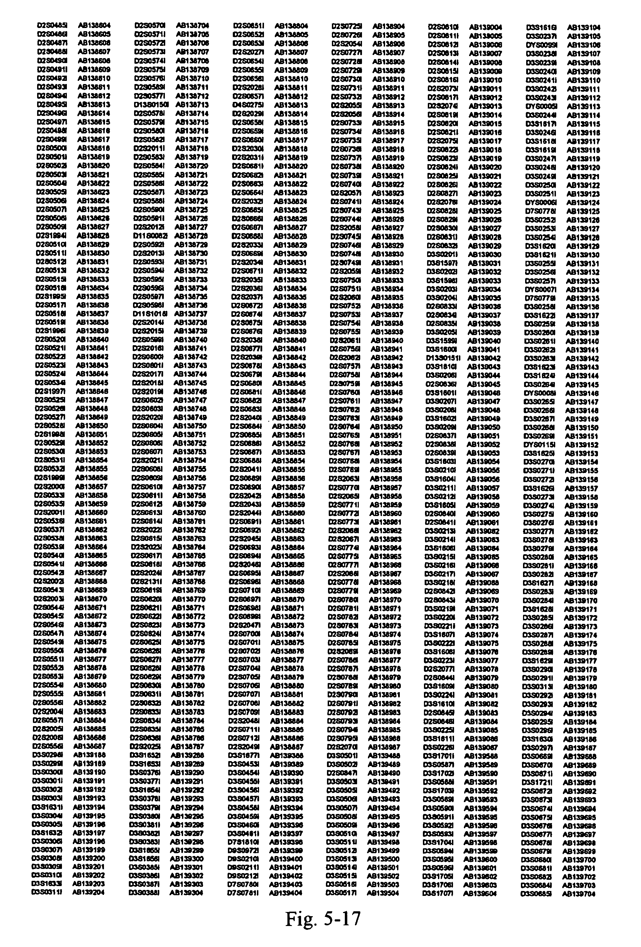

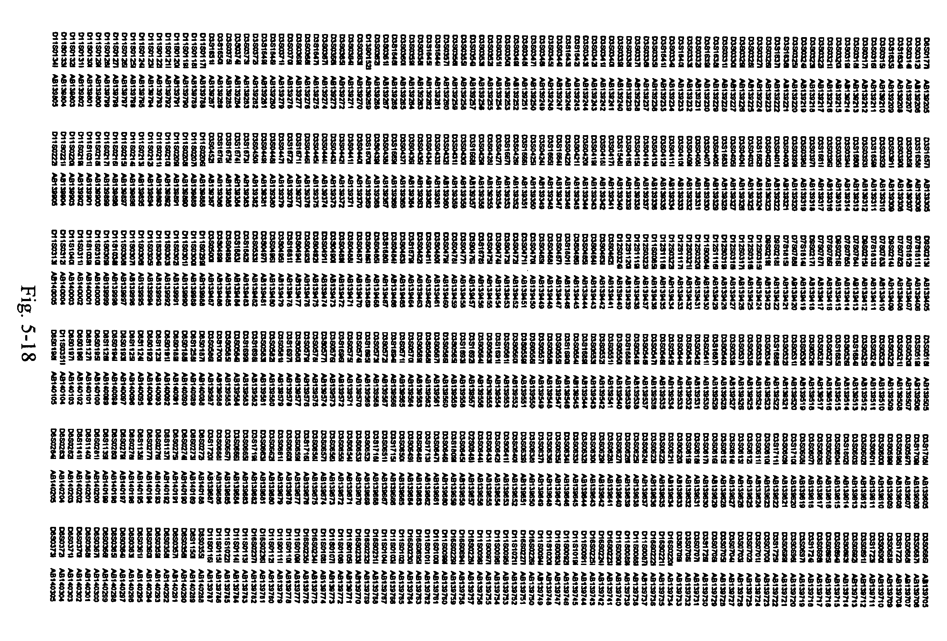

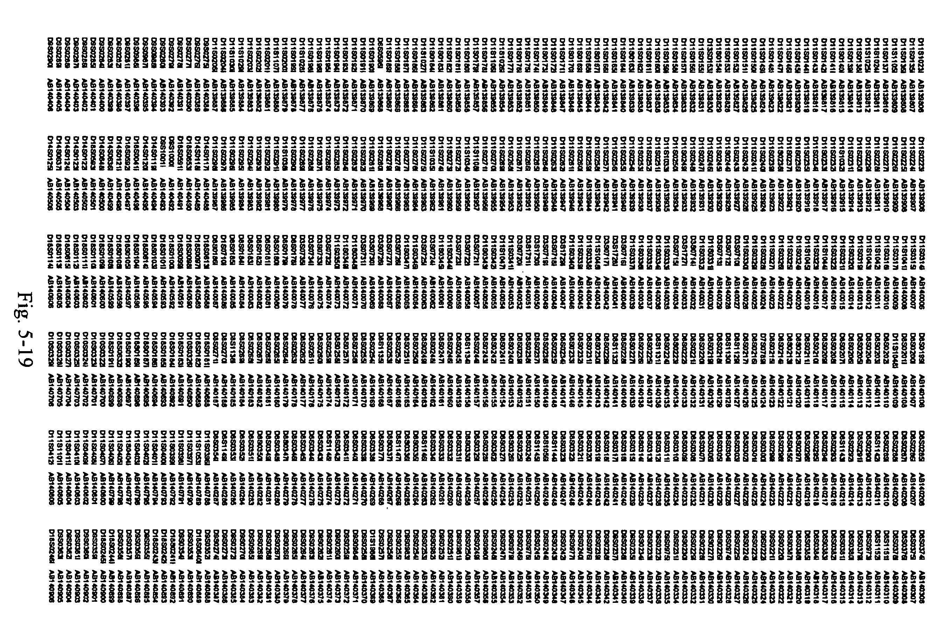

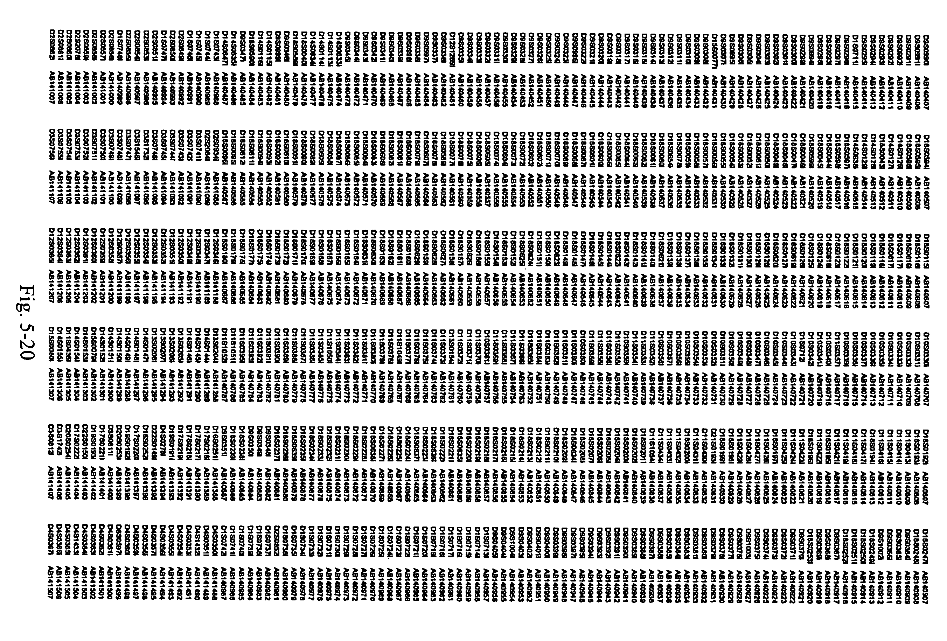

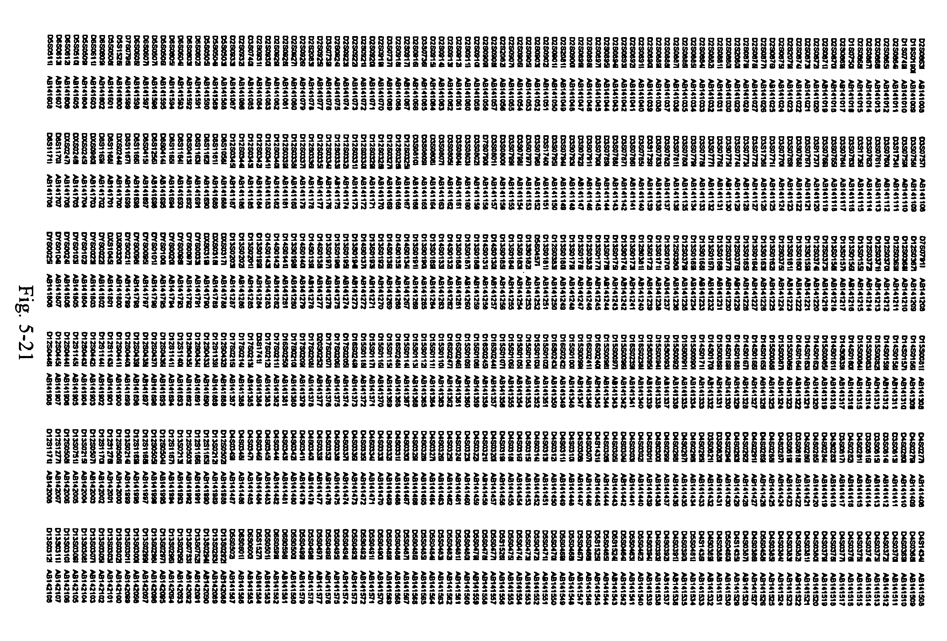

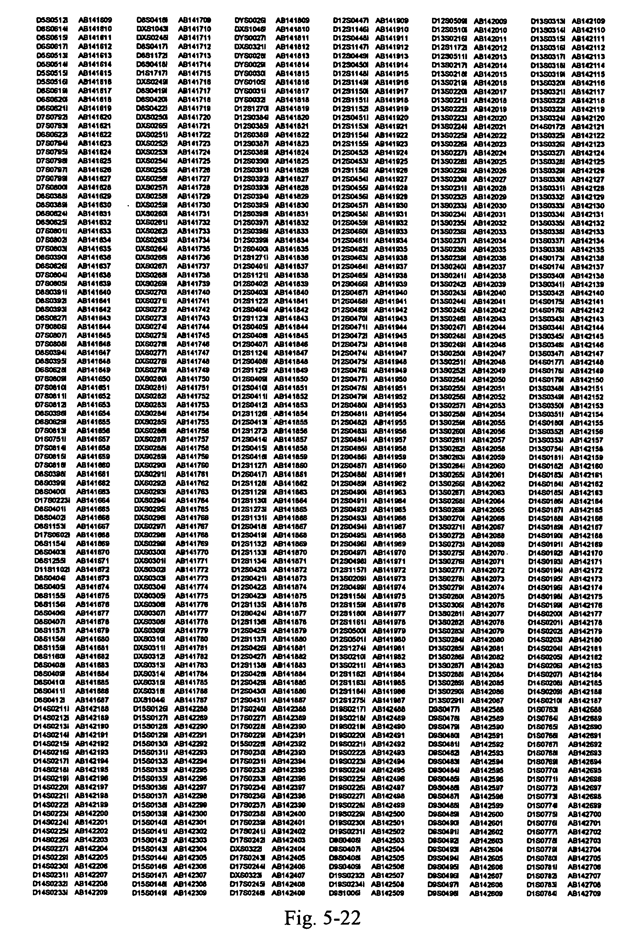

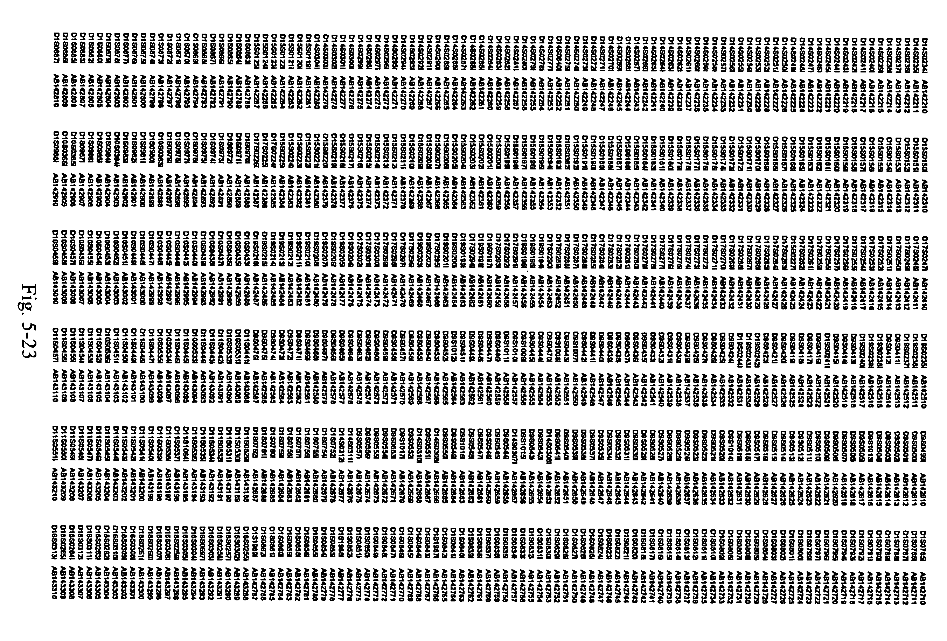

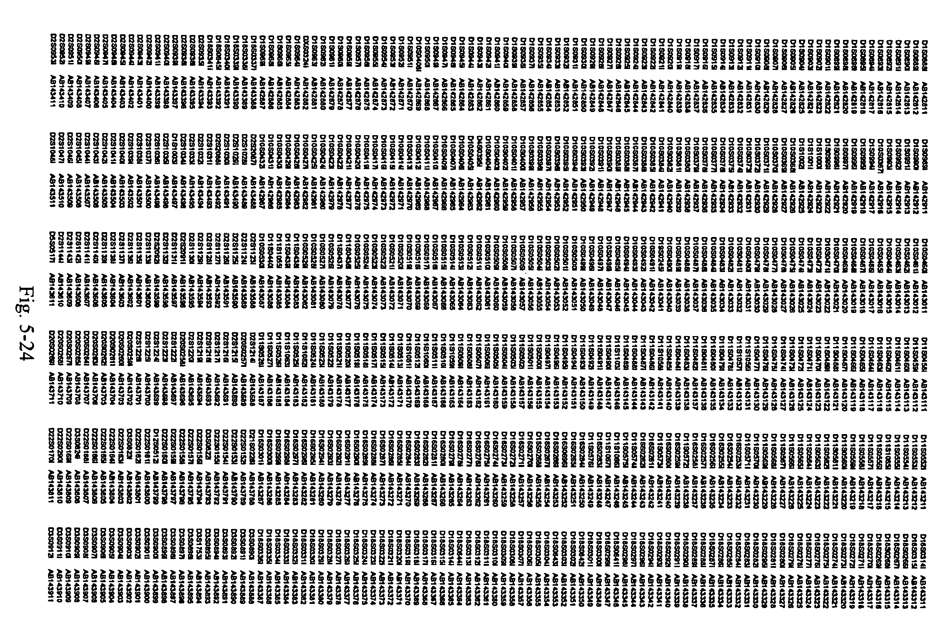

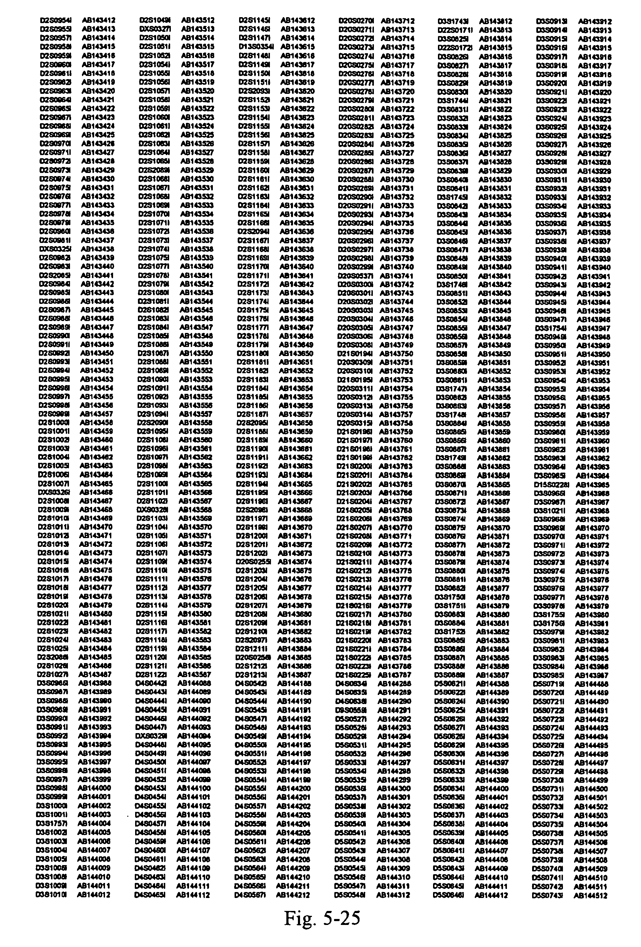

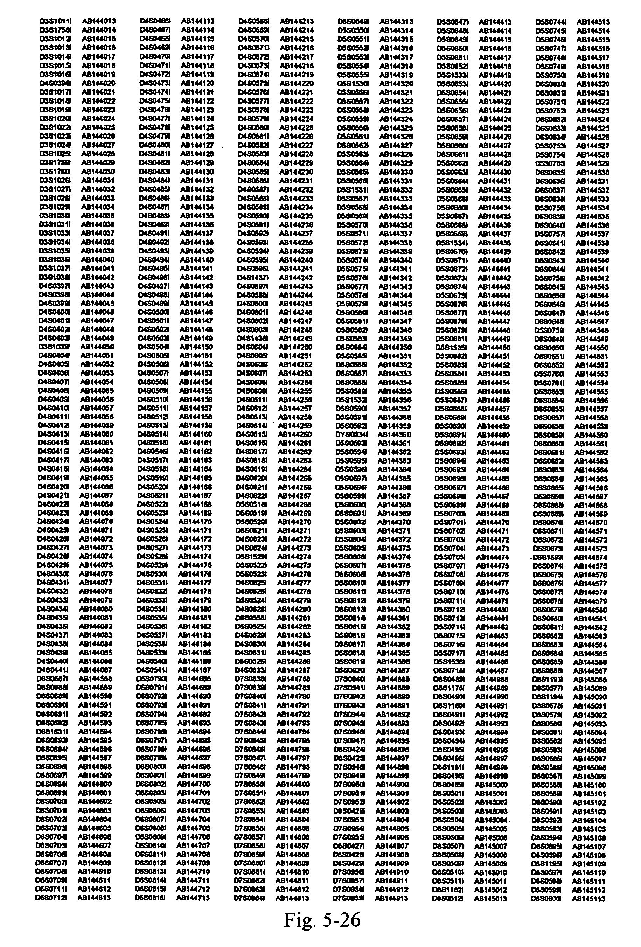

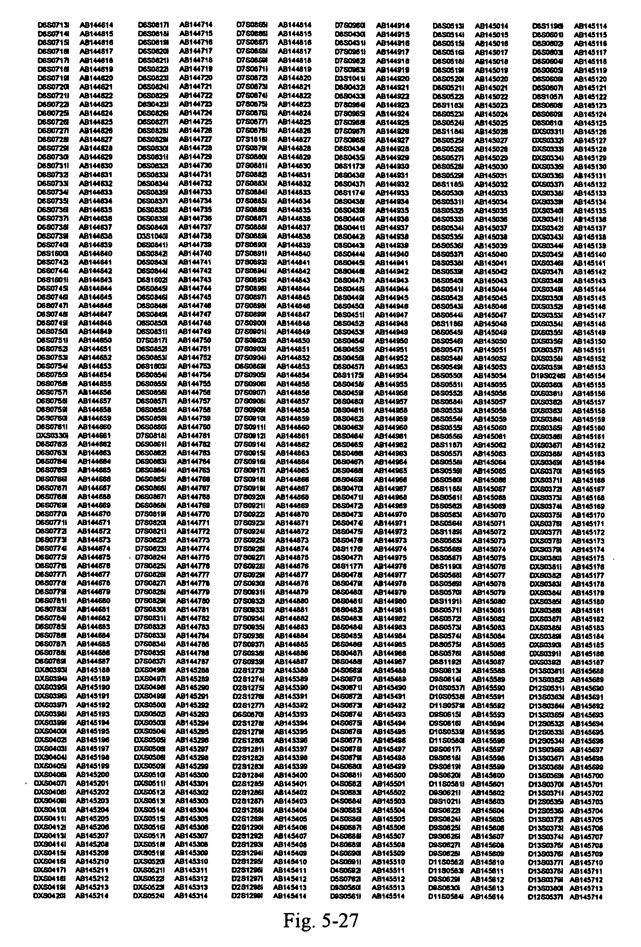

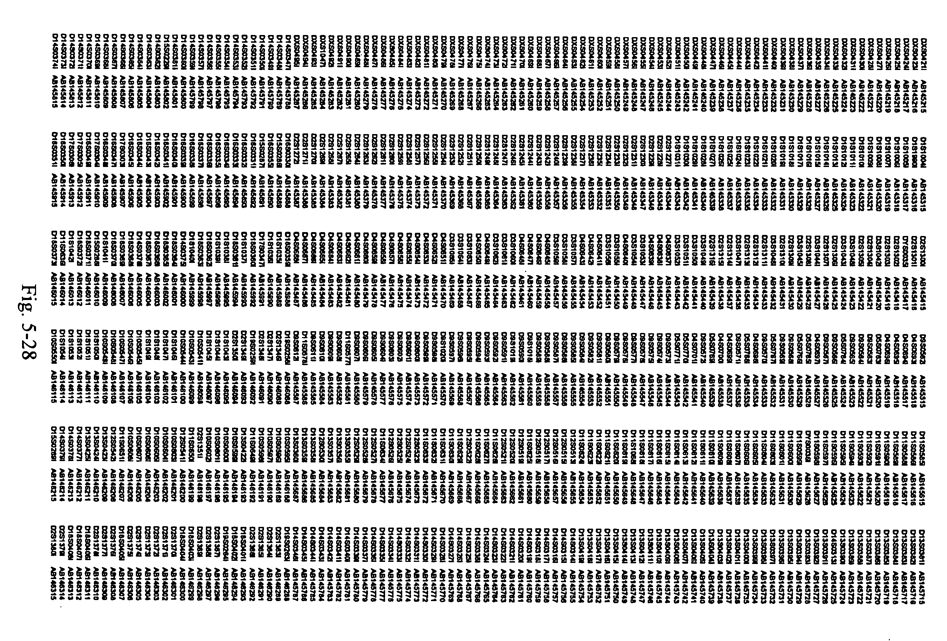

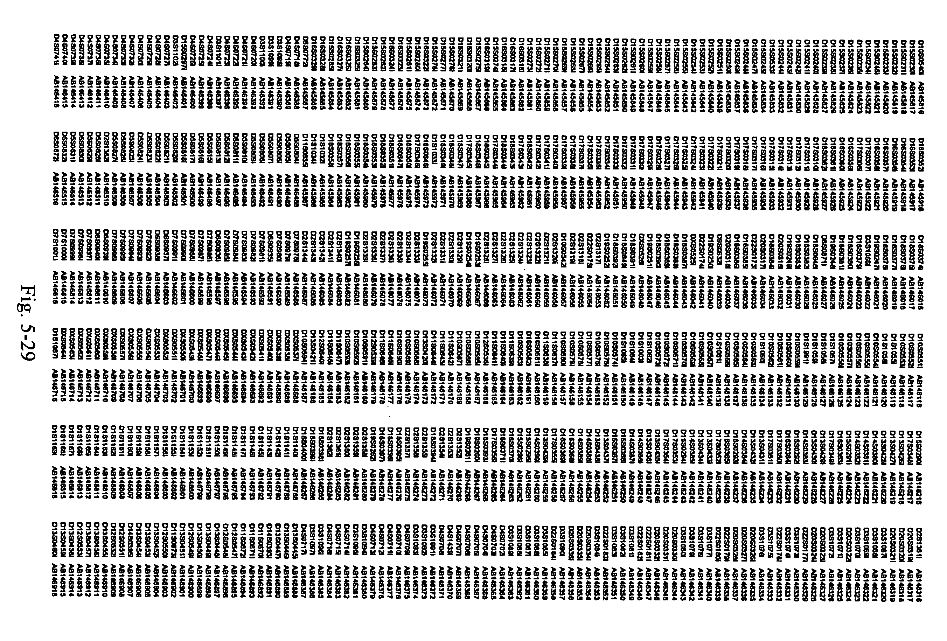

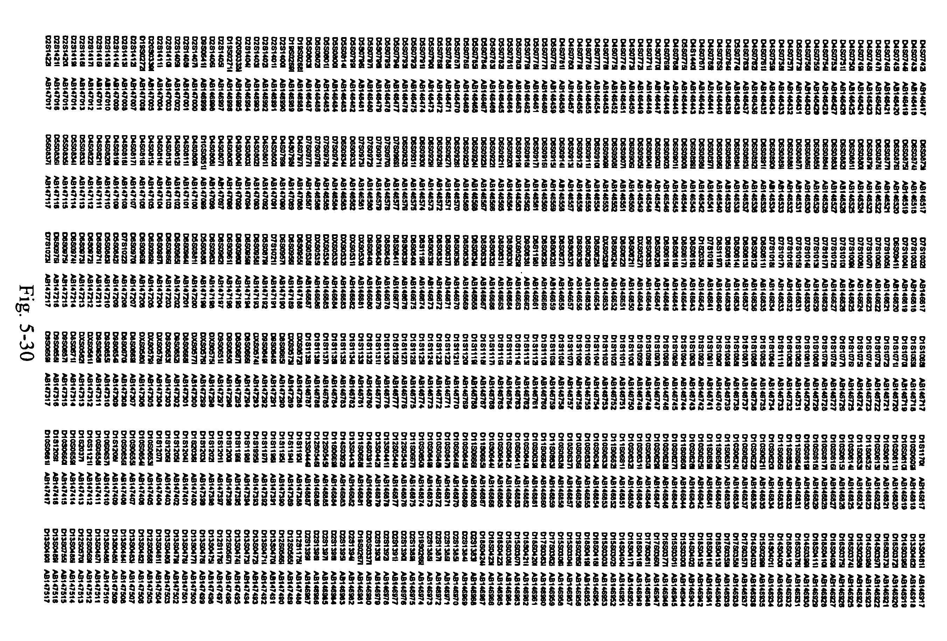

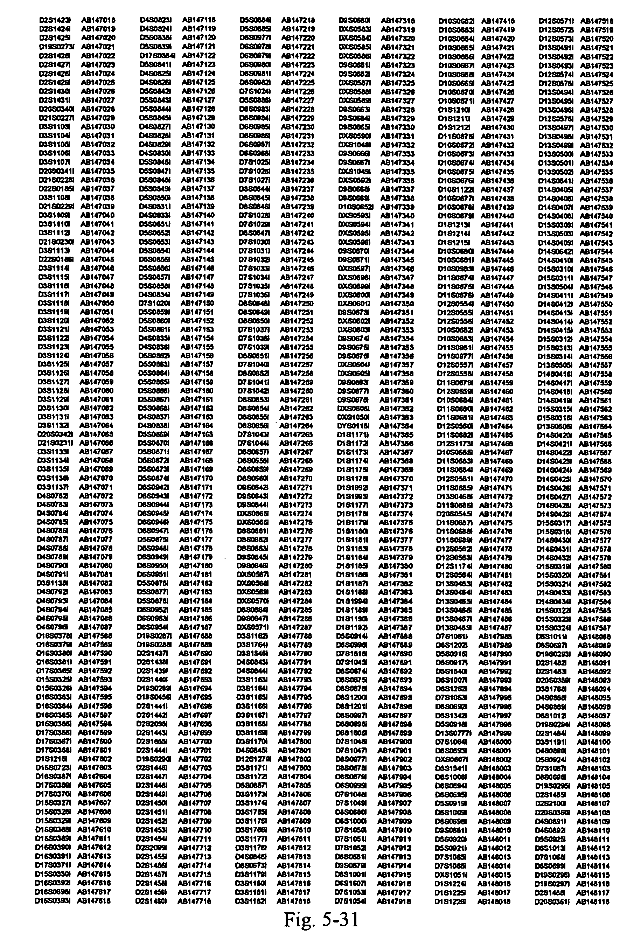

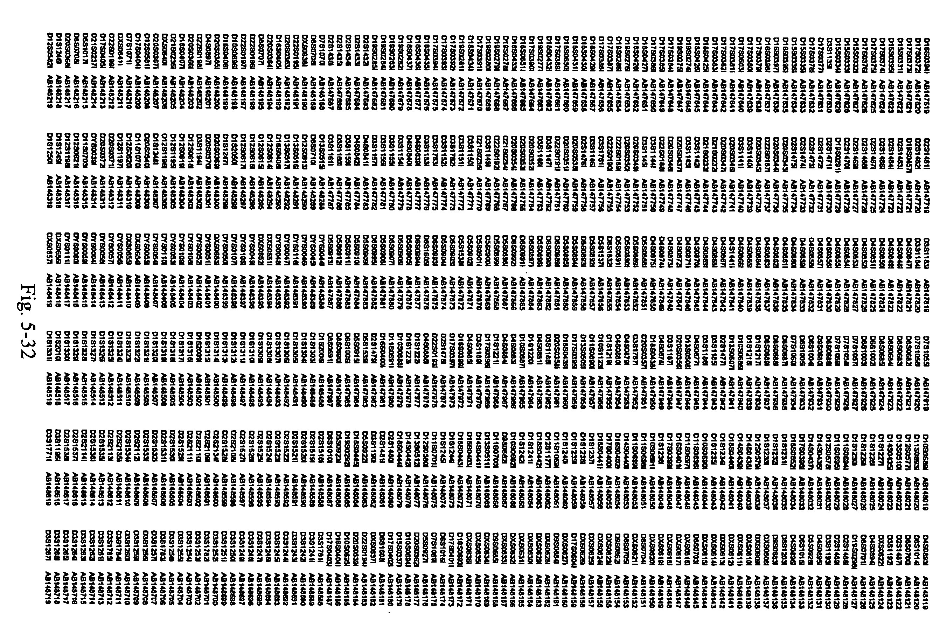

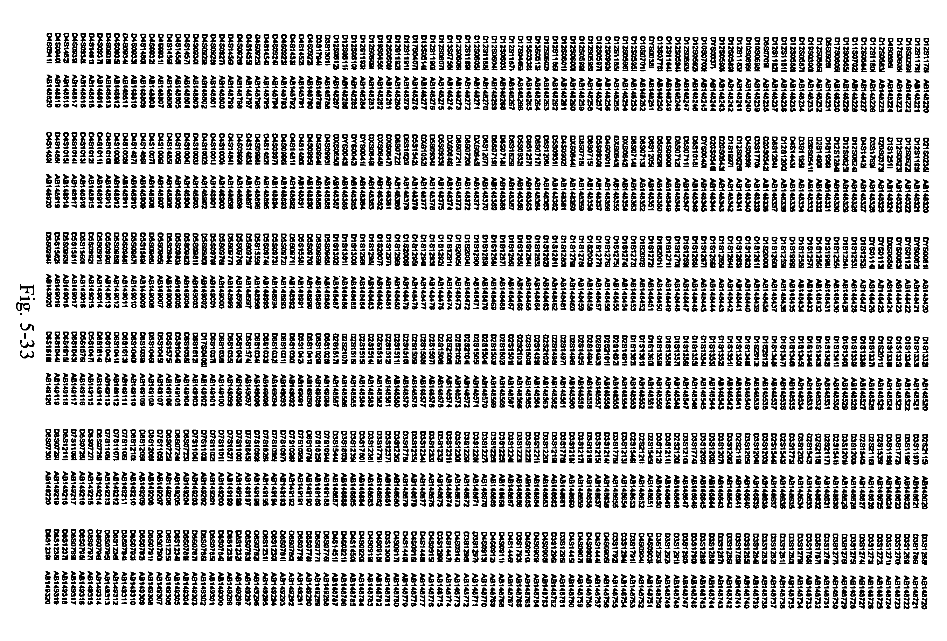

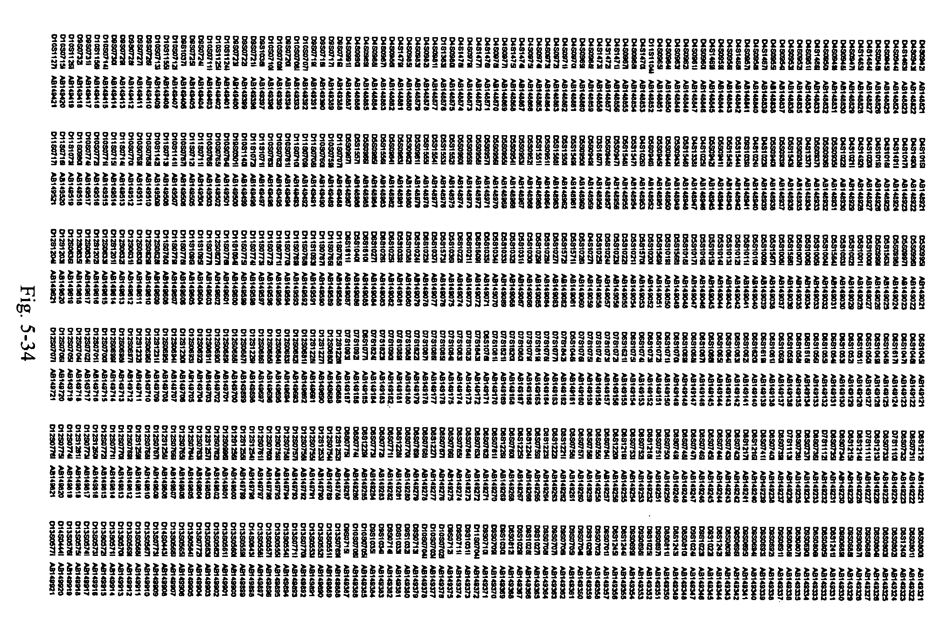

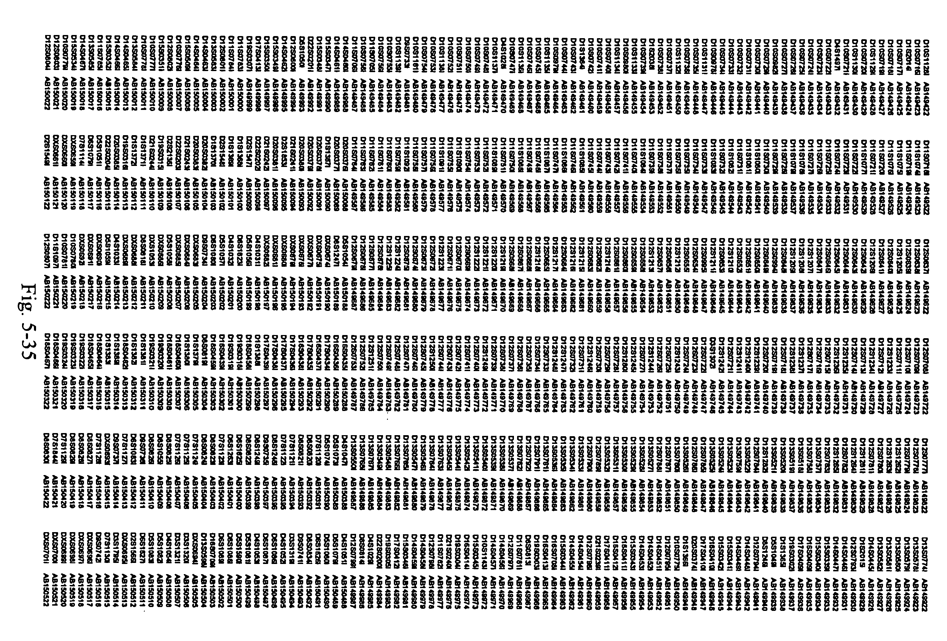

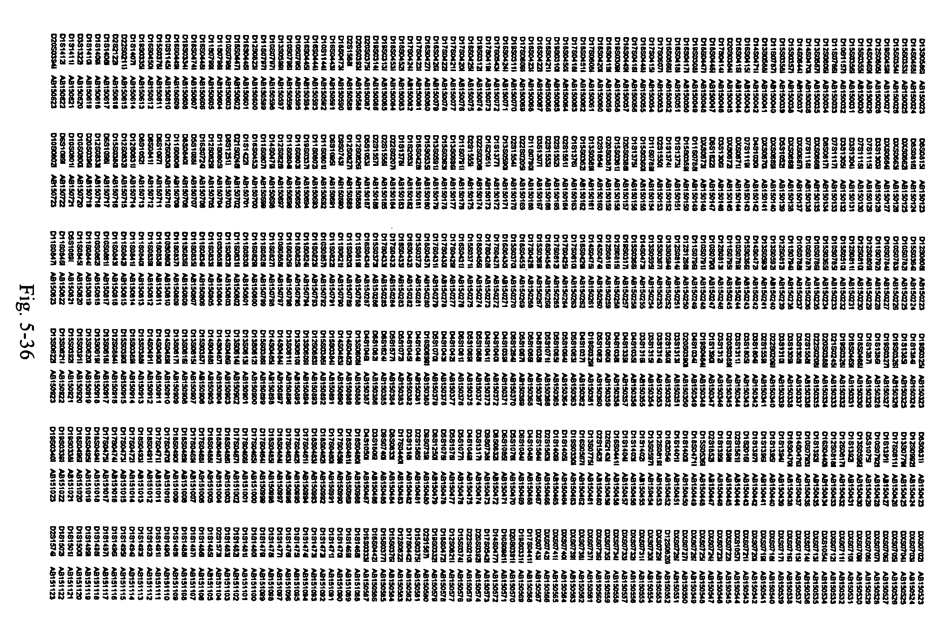

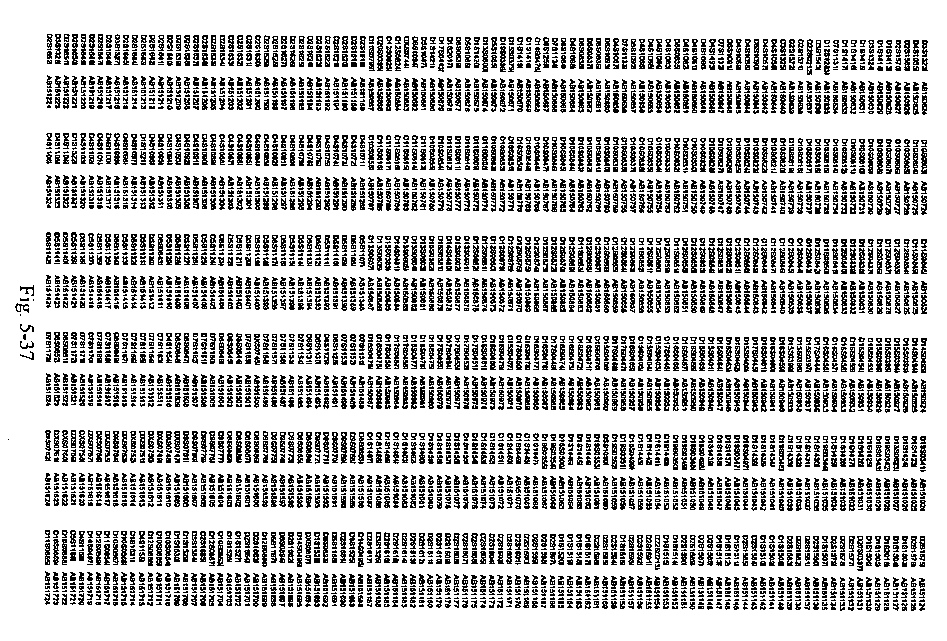

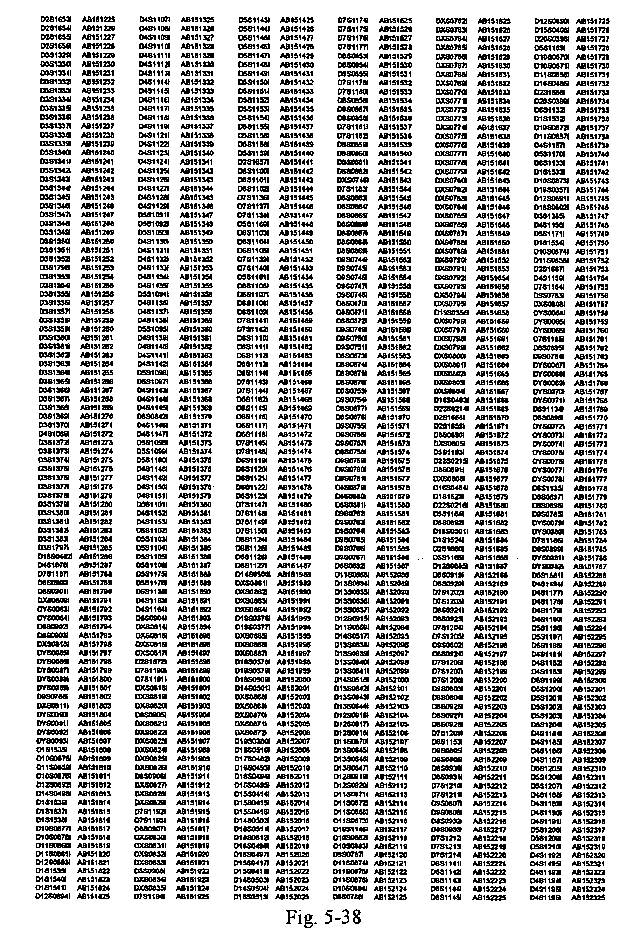

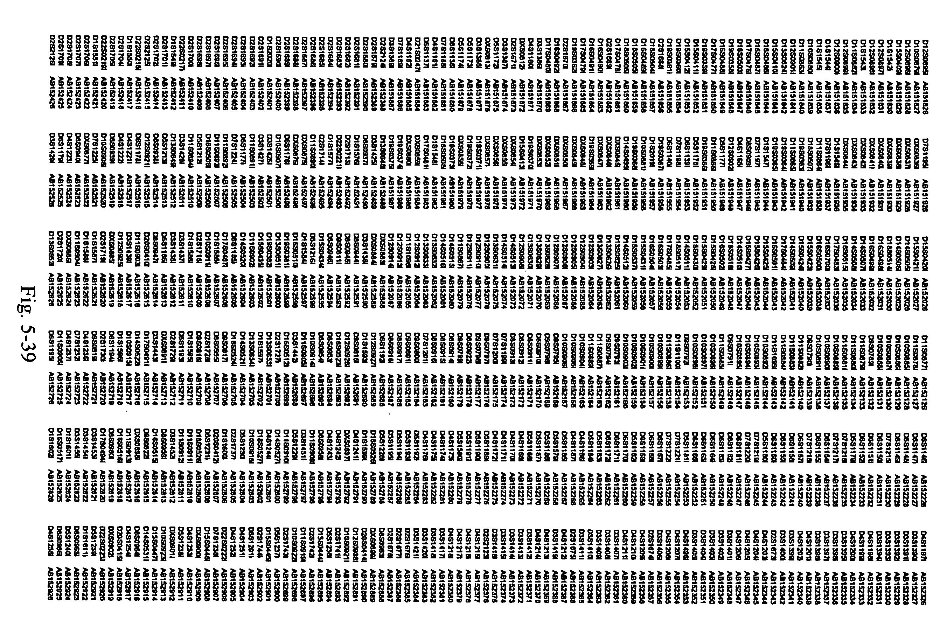

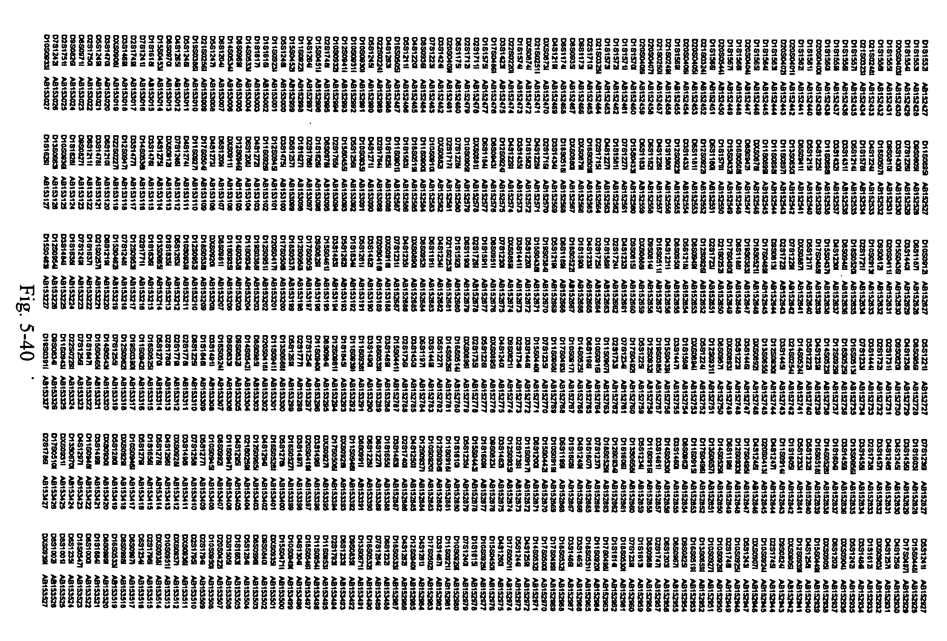

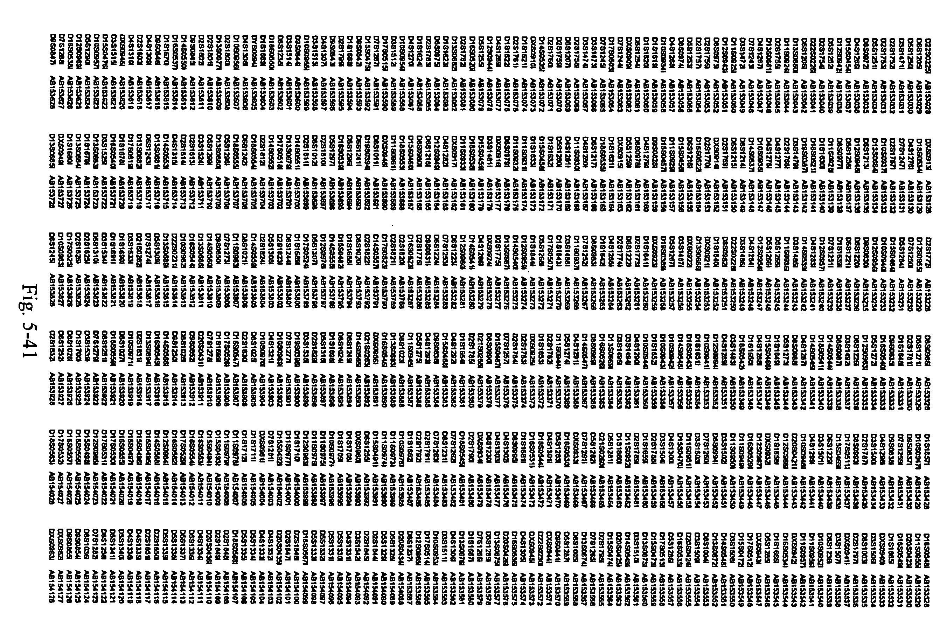

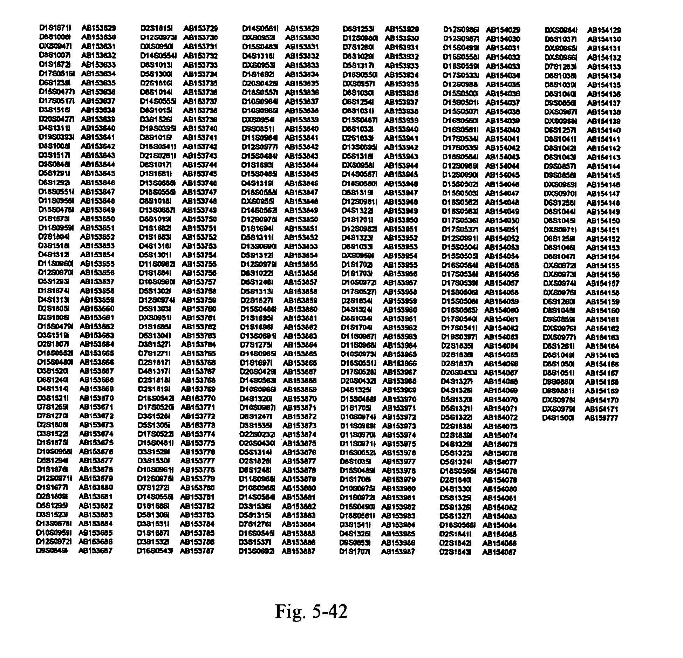

Figure 5-6 is a list showing information on the designations (left in each column) and Genbank registration numbers (right in each column) of microsatellite markers and primers used in the present invention;
Figure 5-7 is a list showing information on the designations (left in each column) and Genbank registration numbers (right in each column) of microsatellite markers and primers used in the present invention;
Figure 5-8 is a list showing information on the designations (left in each column) and Genbank registration numbers (right in each column) of microsatellite markers and primers used in the present invention;
Figure 5-9 is a list showing information on the designations (left in each column) and Genbank registration numbers (right in each column) of microsatellite markers and primers used in the present invention;
Figure 5-10 is a list showing information on the designations (left in each column) and Genbank registration numbers (right in each column) of microsatellite markers and primers used in the present invention;
Figure 5-11 is a list showing information on the designations (left in each column) and Genbank registration numbers (right in each column) of microsatellite markers and primers used in the present invention;
Figure 5-12 is a list showing information on the designations (left in each column) and Genbank registration numbers (right in each column) of microsatellite markers and primers used in the present invention;
Figure 5-13 is a list showing information on the designations (left in each column) and Genbank registration numbers (right in each column) of microsatellite markers and primers used in the present invention;
Figure 5-14 is a list showing information on the designations (left in each column) and Genbank registration numbers (right in each column) of microsatellite markers and primers used in the present invention;
Figure 5-15 is a list showing information on the designations (left in each column) and Genbank registration numbers (right in each column) of microsatellite markers and primers used in the present invention;
Figure 5-16 is a list showing information on the designations (left in each column) and Genbank registration numbers (right in each column) of microsatellite markers and primers used in the present invention;
Figure 5-17 is a list showing information on the designations (left in each column) and Genbank registration numbers (right in each column) of microsatellite markers and primers used in the present invention;
Figure 5-18 is a list showing information on the designations (left in each column) and Genbank registration numbers (right in each column) of microsatellite markers and primers used in the present invention;
Figure 5-19 is a list showing information on the designations (left in each column) and Genbank registration numbers (right in each column) of microsatellite markers and primers used in the present invention;
Figure 5-20 is a list showing information on the designations (left in each column) and Genbank registration numbers (right in each column) of microsatellite markers and primers used in the present invention;
Figure 5-21 is a list showing information on the designations (left in each column) and Genbank registration numbers (right in each column) of microsatellite markers and primers used in the present invention;
Figure 5-22 is a list showing information on the designations (left in each column) and Genbank registration numbers (right in each column) of microsatellite markers and primers used in the present invention;
Figure 5-23 is a list showing information on the designations (left in each column) and Genbank registration numbers (right in each column) of microsatellite markers and primers used in the present invention;
Figure 5-24 is a list showing information on the designations (left in each column) and Genbank registration numbers (right in each column) of microsatellite markers and primers used in the present invention;
Figure 5-25 is a list showing information on the designations (left in each column) and Genbank registration numbers (right in each column) of microsatellite markers and primers used in the present invention;
Figure 5-26 is a list showing information on the designations (left in each column) and Genbank registration numbers (right in each column) of microsatellite markers and primers used in the present invention;
Figure 5-27 is a list showing information on the designations (left in each column) and Genbank registration numbers (right in each column) of microsatellite markers and primers used in the present invention;
Figure 5-28 is a list showing information on the designations (left in each column) and Genbank registration numbers (right in each column) of microsatellite markers and primers used in the present invention;
Figure 5-29 is a list showing information on the designations (left in each column) and Genbank registration numbers (right in each column) of microsatellite markers and primers used in the present invention;
Figure 5-30 is a list showing information on the designations (left in each column) and Genbank registration numbers (right in each column) of microsatellite markers and primers used in the present invention;
Figure 5-31 is a list showing information on the designations (left in each column) and Genbank registration numbers (right in each column) of microsatellite markers and primers used in the present invention;
Figure 5-32 is a list showing information on the designations (left in each column) and Genbank registration numbers (right in each column) of microsatellite markers and primers used in the present invention;
Figure 5-33 is a list showing information on the designations (left in each column) and Genbank registration numbers (right in each column) of microsatellite markers and primers used in the present invention;
Figure 5-34 is a list showing information on the designations (left in each column) and Genbank registration numbers (right in each column) of microsatellite markers and primers used in the present invention;
Figure 5-35 is a list showing information on the designations (left in each column) and Genbank registration numbers (right in each column) of microsatellite markers and primers used in the present invention;
Figure 5-36 is a list showing information on the designations (left in each column) and Genbank registration numbers (right in each column) of microsatellite markers and primers used in the present invention;
Figure 5-37 is a list showing information on the designations (left in each column) and Genbank registration numbers (right in each column) of microsatellite markers and primers used in the present invention;
Figure 5-38 is a list showing information on the designations (left in each column) and Genbank registration numbers (right in each column) of microsatellite markers and primers used in the present invention;
Figure 5-39 is a list showing information on the designations (left in each column) and Genbank registration numbers (right in each column) of microsatellite markers and primers used in the present invention;
Figure 5-40 is a list showing information on the designations (left in each column) and Genbank registration numbers (right in each column) of microsatellite markers and primers used in the present invention;
Figure 5-41 is a list showing information on the designations (left in each column) and Genbank registration numbers (right in each column) of microsatellite markers and primers used in the present invention; and
Figure 5-42 is a list showing information on the designations (left in each column) and Genbank registration numbers (right in each column) of microsatellite markers and primers used in the present invention.

### Best Mode for Carrying Out the Invention

A gene mapping method used in the present invention is a method described in the Patent Document 1. Specifically, this method comprises: using forward and reverse primers corresponding to each DNA sequence of consecutive DNA sequences comprisingMS polymorphic markers assigned at given intervals, preferably approximately 100-kb intervals, on the human genome to amplify the DNA sequence samples by polymerase chain reaction PCR; performing electrophoresis on a high resolution gel such as a DNA sequencer; and measuring and analyzing the microsatellite polymorphic marker-containing DNA sequence fragments, which are amplification products.

MS polymorphic markers exhibiting false positive can be decreased drastically without forced correction by adopting multi-phased screening that involves performing a first (first-phase) screening using forward and reverse primers corresponding to MS polymorphic markers assigned genomewide and performing a second (second-phase) screening on MS polymorphic markers exhibiting positive in the first screening by use of a different sample population.

The position of a target gene is restricted by the multi-phased screening using MS. Then, candidate regions or gene loci can further be determined in detail by another gene mapping method. For example, analysis using SNP is effective forthispurpose. Specifically, thepolymorphismfrequencies of SNPs in the candidate regions that appear to have the target gene are compared, for example by association analysis, between populations of patients and normal individuals, andSNPmarkers with linkage disequilibrium detected by haplotype analysis can be detected by linkage disequilibrium analysis.

To identify RA susceptibility genes, the present invention adopted a previously reported pooled DNA method as a screening method with good cost efficiency using 27, 158 MS markers including 20,755 newly established loci. The genome association analysis was conducted by a three-phased screening method involving three major steps as described above: (1) three-phased genomic screening for reducing a type I error rate; (2) the confirmation of association of pools by individual genotyping on positive MS loci; and (3) identification by detailed individual genotyping on SNP markers in the neighborhoods of candidate regions in screened and additional populations.

The association analysis of the whole genome demonstrated the strongest association of the HLA-DRB1 gene, which has previouslybeen known to have association with RA (P=9.7×10⁻²⁰). Furthermore, strong association was observed, independently of HLA-DRB1, inNOTCH4 (P=1.1×10⁻¹¹) andTNXB (P=7.6×10⁻⁷) genes on chromosome 6 also carrying HLA-DRB1. Moreover, novel association was found in a mitochondrial-related gene cluster on 11q13.4 containing mitochondrial ribosomal protein L48 (MRPL48) and two mitochondrial proteins called uncoupling proteins (UCP2 and UCP3). Weak association was seen on 10p13 and 14q23.1. In addition to these novel associations, association was confirmed in IkBL (Non-Patent Document 4) and PADI4 (Non-Patent Document 5) genes, which have already been reported to have association with RA, as with HLA-DRB1.

Namely, statistically significant difference in allele frequencies of SNPs present in TNXB, NOTCH4, RAB6A, MPRL48, UCP2, and UCP3 genes found de novo to have association was observed between RA patients and normal individuals. Thus, a consecutive partial DNA sequence comprising at least one base exhibiting signal nucleotide polymorphism present in any of these gene regions or a complementary strand of the partial DNA sequence can be utilized as a marker gene for arthrorheumatism test.

Specifically, it is preferred that the base exhibiting single nucleotide polymorphism should be selected from the group consisting of:
the 61st base in SEQ ID NO: 1 or a corresponding base on a complementary strand thereof;
the 61st base in SEQ ID NO: 2 or a corresponding base on a complementary strand thereof;
the 61st base in SEQ ID NO: 3 or a corresponding base on a complementary strand thereof;
the 61st base in SEQ ID NO: 4 or a corresponding base on a complementary strand thereof;
the 401st base in SEQ ID NO: 5 or a corresponding base on a complementary strand thereof;
the 495th base in SEQ ID NO: 6 or a corresponding base on a complementary strand thereof;
the 61st base in SEQ ID NO: 7 or a corresponding base on a complementary strand thereof;
the 61st base in SEQ ID NO: 8 or a corresponding base on a complementary strand thereof;
the 61st base in SEQ ID NO: 9 or a corresponding base on a complementary strand thereof;
the 61st base in SEQ ID NO: 10 or a corresponding base on a complementary strand thereof;
the 401st base in SEQ ID NO: 11 or a corresponding base on a complementary strand thereof;
the 401st base in SEQ ID NO: 12 or a corresponding base on a complementary strand thereof;
the 401st base in SEQ ID NO: 13 or a corresponding base on a complementary strand thereof;
the 503rd base in SEQ ID NO: 14 or a corresponding base on a complementary strand thereof;
the 201st base in SEQ ID NO: 15 or a corresponding base on a complementary strand thereof;
the 511th base in SEQ ID NO: 16 or a corresponding base on a complementary strand thereof;
the 201st base in SEQ ID NO: 17 or a corresponding base on a complementary strand thereof;
the 51st base in SEQ ID NO: 18 or a corresponding base on a complementary strand thereof;
the 61st base in SEQ ID NO: 19 or a corresponding base on a complementary strand thereof;
the 497th base in SEQ ID NO: 20 or a corresponding base on a complementary strand thereof;
the 201st base in SEQ ID NO: 21 or a corresponding base on a complementary strand thereof; and
the 201st base in SEQ ID NO: 22 or a corresponding base on a complementary strand thereof.

SEQ ID NOs: 1 to 5 represent partial sequences of the TNXB gene, SEQ ID NOs: 6 to 13 represent partial sequences of the NOTCH4 gene, SEQ ID NO: 14 represents a partial sequence of the RAB6A gene, SEQ ID NOs: 15 to 18 represent partial sequences of the MPRL48 gene, SEQ ID NOs: 19 and 20 represent partial sequences of the FLJ11848 gene, SEQ IDNO: 21 represents a partial sequence of the UCP2 gene, and SEQ ID NO: 22 represents a partial sequence of UCP3.

These marker genes can be used in genetic testing on RA.

For example, the consecutive DNA sequence comprising the base exhibiting single nucleotide polymorphism is amplified, for example by PCR, using forward and reverse primers positioned to keep the base exhibiting single nucleotide polymorphism in between them. Nucleotide sequences of the obtained DNA fragments can be determined and compared with a determined corresponding nucleotide sequence from a normal individual to thereby test the presence or absence of a genetic factor for RA.

The forward primer used in the test is a primer having the same nucleotide sequence as a sequence extending in the 3'-end direction from the 5' end of the DNA sequence of the marker gene containing the base exhibiting single nucleotide polymorphism, which has been mapped on the human genome, and includes those of 15 to 100 bases, preferably 15 to 25 bases, more preferably 18 to 22 bases, in length. The reverse primer is a primer having a nucleotide sequence complementary to a sequence extending in the 5'-end direction from the 3' end of the DNA sequence of the marker gene, and those of 15 to 100 bases, preferably 15 to 25 bases, more preferably 18 to 22 bases, in length can be used as the reverse primer.

Examples of primers for amplifying the marker genes having the DNA sequences of SEQ ID NOs : 1 to 22 include those having DNA sequences represented by SEQ ID NOs: 23 to 66. The relationship of their correspondence is as follows:

| Marker gene | Forward primer | Reverse primer |
|---|---|---|
| SEQ ID NO: 1 | SEQ ID NO: 23 | SEQ ID NO: 24 |
| SEQ ID NO: 2 | SEQ ID NO: 25 | SEQ ID NO: 26 |
| SEQ ID NO: 3 | SEQ ID NO: 27 | SEQ ID NO: 28 |
| SEQ ID NO: 4 | SEQ ID NO: 29 | SEQ ID NO: 30 |
| SEQ ID NO: 5 | SEQ ID NO: 31 | SEQ ID NO: 32 |
| SEQ ID NO: 6 | SEQ ID NO: 33 | SEQ ID NO: 34 |
| SEQ ID NO: 7 | SEQ ID NO: 35 | SEQ ID NO: 36 |
| SEQ ID NO: 8 | SEQ ID NO: 37 | SEQ ID NO: 38 |
| SEQ ID NO: 9 | SEQ ID NO: 39 | SEQ ID NO: 40 |
| SEQ ID NO: 10 | SEQ ID NO: 41 | SEQ ID NO: 42 |
| SEQ ID NO: 11 | SEQ ID NO: 43 | SEQ ID NO: 44 |
| SEQ ID NO: 12 | SEQ ID NO: 45 | SEQ ID NO: 46 |
| SEQ ID NO: 13 | SEQ ID NO: 47 | SEQ ID NO: 48 |
| SEQ ID NO: 14 | SEQ ID NO: 49 | SEQ ID NO: 50 |
| SEQ ID NO: 15 | SEQ ID NO: 51 | SEQ ID NO: 52 |
| SEQ ID NO: 16 | SEQ ID NO: 53 | SEQ ID NO: 54 |
| SEQ ID NO: 17 | SEQ ID NO: 55 | SEQ ID NO: 56 |
| SEQ ID NO: 18 | SEQ ID NO: 57 | SEQ ID NO: 58 |
| SEQ ID NO: 19 | SEQ ID NO: 59 | SEQ ID NO: 60 |
| SEQ ID NO: 20 | SEQ ID NO: 61 | SEQ ID NO: 62 |
| SEQ ID NO: 21 | SEQ ID NO: 63 | SEQ ID NO: 64 |
| SEQ ID NO: 22 | SEQ ID NO: 65 | SEQ ID NO: 66 |

Alternatively, the presence or absence of an inheriting factor for RA can also be examined by using the marker genes of the present invention as probes to screen a DNA sample from a test subject, determining a nucleotide sequence of the obtained DNA of the test subject, and then comparing the sequence with a sequence from a normal individual.

In this context, the probe used may be the marker gene of the present invention itself or may be the consecutive DNA sequence comprising the base exhibiting single nucleotide polymorphismpresent in the marker gene, a complementary strand thereof, or sequences hybridized by them. Preferably, a probe of 15 to 100 bases, preferably 15 to 25 bases, more preferably 18 to 22 bases, in length can be used.

On the other hand, coding regions encoded by the RA susceptibility genes can be determined by determining the full-length nucleotide sequences of the RA susceptibility genes on the basis of TNXB, NOTCH4, RAB6A, MPRL48, UCP2 and UCP3 genes found de novo to have association. As a result, amino acid sequences of proteins encoded by the genes can be identified. Since proteins with these amino acid sequences are highly likely to participate in RA pathogenesis or onset mechanisms, RA can be prevented or treated by promoting or inhibiting the functions of these proteins.

Thus, the present invention also relates to a screening method using the proteins. Substances promoting or isolating the functions of the proteins, that is, agonists or antagonists can be identified by the screening method. The antagonist used herein encompasses not only chemical small molecules but also biologically relevant substances such as antibodies, antibody fragments, and antisense oligonucleotides. These agonists or antagonists are effective as diagnostic, preventive, and/or therapeutic drugs for RA.

The protein described above may be produced in a transformed cell obtained by preparing a vector comprising a DNA sequence containing at least the coding region of any of the marker genes identified by the present invention, and then transforming the vector into an appropriate host cell.

### Example

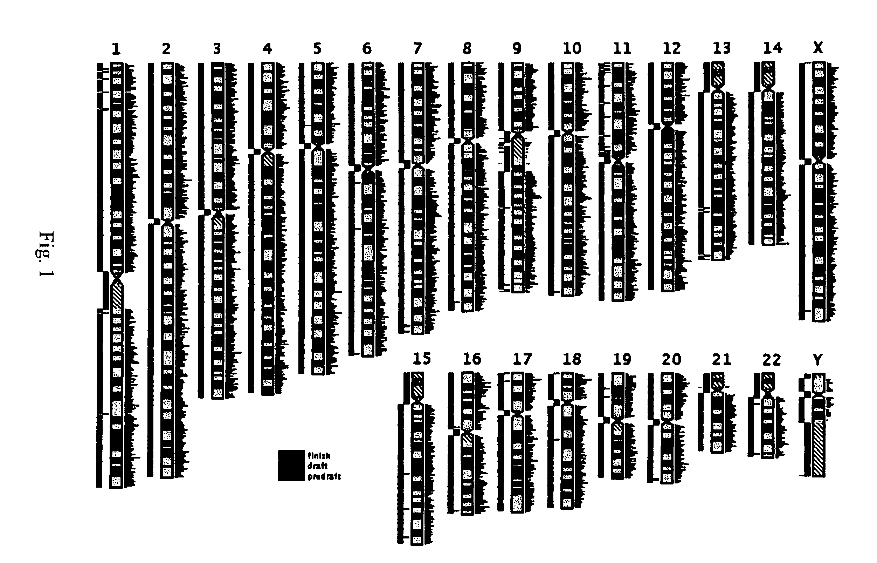

Microsatellite (MS) Detection and PCR Primer Design:
MS sequences with 2-, 3-, 4-, 5-, or 6-base repeat units were detected with Apollo program applicable to Sputnik in four versions of the human genome draft sequences from Golden Path Oct. 2000 to NCBI build 30. PCR primers for amplifying these repeats under single reaction conditions were automatically designed with Discover program applicable to Primer Express. Topreventdifferential amplification, these PCR primers were designed to contain no SNP in their sequences (Sham et al, 2002).
A pattern with a number of peaks exhibiting the polymorphisms of MS markers in a pool of Japanese (Barcellos. L. F. et al., Am. J. Hum. Genet., 61, 734 (1997)) was compared with that of European pools. As a result, individual polymorphic MS markers in the Japanese pool exhibited a different pattern from that of two European pools (data not shown). The result of the comparison between the races showed that the pattern with a number of peaks in the Japanese pool reflects polymorphism in MS length and is not experimental error.
In the present invention, 27, 158 polymorphic MS markers were assigned and mapped on the human draft sequence (NCBI build 30) (Figure 1). Among these markers, 20,755 markers were assigned de novo by the present inventors, while remaining 6,403 were known markers such as Genethon and CHLC markers. The average heterozygosity and average allele number of 27, 039 markers except for 119 markers mapped on the Y gene were 0.67±0.16 and 6.4±3.1, respectively. The average marker spacing thereof was 108.1 kb (SD=64.5 kb; max=930.1 kb) (see Table 1). These markers can detect linkage disequilibrium up to approximately 50 kb distant from a disease locus at a rough estimate. Accordingly, these markers were used to conduct case-control association analysis on RA. Those 27,039 microsatellites and primer sequences used for their amplification were deposited in Genbank as registration numbers listed in Figure 5.

**[Table 1]**

| **Supplementary Table 1. Microsatellite marker spacing** | | | |
|---|---|---|---|
| **Chromosome** | **Spacing (kb)** | | |
| | **Average** | **SD** | **Max.** |
| **1** | **104.7** | **64.5** | **581.7** |
| **2** | **103.6** | **61.2** | **521.4** |
| **3** | **103.0** | **67.0** | **766.7** |
| **4** | **113.0** | **68.4** | **522.4** |
| **5** | **107.2** | **61.9** | **461.8** |
| **6** | **108.6** | **58.3** | **428.5** |
| **7** | **100.2** | **62.4** | **634.6** |
| **8** | **108.3** | **68.6** | **587.3** |
| **9** | **106.4** | **65.5** | **930.1** |
| **10** | **106.6** | **62.6** | **510.2** |
| **11** | **104.8** | **64.6** | **463.0** |
| **12** | **106.8** | **66.6** | **674.2** |
| **13** | **108.3** | **57.4** | **356.6** |
| **14** | **115.9** | **52.6** | **350.2** |
| **15** | **119.1** | **67.4** | **625.9** |
| **16** | **114.1** | **77.3** | **526.0** |
| **17** | **112.1** | **70.9** | **546.4** |
| **18** | **106.5** | **63.6** | **563.7** |
| **19** | **110.8** | **69.9** | **421.8** |
| **20** | **108.6** | **56.9** | **337.0** |
| **21** | **108.2** | **58.0** | **378.7** |
| **22** | **120.3** | **68.1** | **505.7** |
| **X** | **123.5** | **65.5** | **443.5** |
| **Total** | **108.1** | **64.5** | **930.1** |

| | | | |
|---|---|---|---|
| Microsatellite markers were mapped on the NCBI build 30. | | | |

In the present invention, 940 test subjects with RA (case population) and the same number of normal test subjects (control population) were adopted. By permission of the ethical committee of each organization associated with the present invention, informed consent was obtained from each test subject in the case and control populations used in this analysis. RA phenotypes were determined according to American Rheumatism Association diagnostic criteria for RA. All personal data associated with medical information and blood samples were carefully discarded in organization which collected them.

Average age at disease onset in the case population was 47.7±13.1 years old, with the sex ratio of 1:4 (male:female). The average age and sex ratio of the case and control populations were set as equally as possible. The sexes of all samples involved were confirmed by amelogenin (enamel protein) genotyping (Akane, A., et al., Forensic Sci. Int., 49, 81 (1991)). Preliminary PCR test for checking DNA levels was conducted by PCR direct sequencing as previously reported (Voorter, C. E. et al., TissueAntigens, 49, 471 (1997)), while HLA-DRB1 genotypes were examined.

### DNA Sample Preparation and Typing:

DNA was extracted with QIAamp DNA blood kit (QIAGEN) from the sample of each test subject in the populations under standardized conditions for preventing variations in DNA level. Subsequently, to check DNA degradation and RNA contamination, 0.8% agarose gel electrophoresis was performed. After optical density measurement for checking protein contamination, the DNA concentration was determined by three measurements using PicoGreen fluorescence assay (Molecular Probes) as previously described (Collins, H. E. et al., Hum. Genet., 106, 218 (2000)). The standardized pipetting and dispensation of the DNA samples were performed with robots such as Biomek 2000 and Multimek 96 (Beckman).

The pooled DNA template for typing two groups of approximately 30, 000 MS markers was prepared simultaneously with or immediately after the DNA quantification. The pooled DNA level was further tested by comparing allelic distribution between individuals and pooled typing results using three MS markers. After this test, approximately 30, 000 PCR reaction mixtures containing all the components except for the PCR primers were prepared and subsequently dispensed to 96-well PCR reaction plates, followed by storage until use.

After PCR reaction, pooled MS typing and individual genotyping were conducted according to standard protocols using ABI3700 DNA analyzer (Applied Biosystems). The pooled DNA typing could maintain constant accuracy throughout the experiment by using the standardized preparation method. Various data such as peak positions and heights were automatically read by the PickPeak and MultiPeaks programs developed by Applied Biosystems Japan, from the multipeak pattern in the chromatograph files, that is, ABI fsa files.

### Three-Phased Genome Screening by Pooled DNA Method:

A population of 375 individuals with RA (case) and the same number of unaffected individuals (control) were equally divided into three pairs of case and control populations (125 individuals each). Population stratification test was conducted using 22 randomly selected microsatellites sufficient at least for population stratification according to Pritchard's method ( Pritchard, J. K. and Rosenberg, N. A., Am. J. Hum. Genet., 65, 220 (1999)). The results showed the absence of any significant stratification in either case or control populations (Table 2). The prevention of false association by the population stratification test is very important for late-onset diseases such as RA (Risch 2000) where the collection of internal controls is difficult.

**[Table 2]**

| Supplementary Table 2. Stratification teat among case and control populations | | | | | | | | | | | | | | | | |
|---|---|---|---|---|---|---|---|---|---|---|---|---|---|---|---|---|
| Chr. | Markers | Fisher's exact P values | | | | | | | | | | | | | | |
| | | 1st screening (n=125) | | | 2nd screening (n=125) | | | 3rd screwing (n=125) | | | Additional samples (n=565) | | | Total (n= 940) | | |
| | | 2x2 | # of Allele | 2xm | 2x2 | # of Allele | 2xm | 2x2 | # of Allele | 2xm | 2x2 | # of Allele | 2xm | 2x2 | # of Allele | 2xm |
| 1 | D1S03681 | 0.296 | 6 | 0.780 | 0.113 | 5 | 0.181 | 0.248 | 7 | 0.524 | 0.331 | 8 | 0.939 | 0.093 | 9 | 0.627 |
| 2 | D2S1336 | 0.171 | 8 | 0.555 | 0.070 | 8 | 0.034 | 0.059 | 9 | 0.480 | 0.250 | 10 | 0.585 | 0.453 | 10 | 0.880 |
| 3 | D3S2439 | 0.025 | 9 | 0.351 | 0.015 | 10 | 0.073 | 0.040 | 9 | 0.414 | 0.028 | 11 | 0.503 | 0.194 | 11 | 0.775 |
| 4 | G10243 | 0.387 | 7 | 0.884 | 0.268 | 7 | 0.906 | 0.095 | 7 | 0.233 | 0.076 | 8 | 0.202 | 0.020 | 8 | 0.063 |
| 5 | D5S00291 | 0.857 | 3 | 0.960 | 0.710 | 3 | 0.933 | 0.068 | 5 | 0.172 | 0.186 | 4 | 0.399 | 0.146 | 5 | 0.415 |
| 6 | G10114 | 0.373 | 6 | 0.852 | 0.038 | 6 | 0.272 | 0.282 | 7 | 0.464 | 0.012 | 6 | 0.036 | 0.064 | 8 | 0.255 |
| 7 | D7S1802 | 0.157 | 8 | 0.446 | 0.157 | 9 | 0.664 | 0.258 | 8 | 0.882 | 0.009 | 11 | 0.230 | 0.013 | 12 | 0.232 |
| 8 | HUMUT1239 | 0.022 | 7 | 0.034 | 0.399 | 7 | 0.814 | 0.123 | 7 | 0.466 | 0.098 | 6 | 0.453 | 0.282 | 8 | 0.815 |
| 9 | D9S01471 | 0.372 | 7 | 0.717 | 0.125 | 6 | 0.341 | 0.238 | 7 | 0.279 | 0.156 | 7 | 0.733 | 0.226 | 8 | 0.513 |
| 10 | G08808 | 0.210 | 6 | 0.541 | 0.074 | 7 | 0.123 | 0.123 | 6 | 0.475 | 0.030 | 9 | 0.046 | 0.166 | 10 | 0.220 |
| 11 | D11S06891 | 0.215 | 10 | 0.595 | 0.062 | 10 | 0.124 | 0.553 | 9 | 0.986 | 0.308 | 13 | 0.902 | 0.148 | 13 | 0.699 |
| 12 | G08964 | 0.248 | 8 | 0.288 | 0.071 | 7 | 0.109 | 0.229 | 8 | 0.673 | 0.146 | 9 | 0.920 | 0.333 | 9 | 0.820 |
| 13 | D13S01021 | 0.089 | 6 | 0.476 | 0.135 | 6 | 0.368 | 0.187 | 6 | 0.256 | 0.001 | 7 | 0.016 | 0.002 | 7 | 0.020 |
| 14 | D14S608 | 0.015 | 10 | 0.163 | 0.253 | 8 | 0.600 | 0.340 | 10 | 0.867 | 0.051 | 10 | 0.164 | 0.028 | 10 | 0.114 |
| 15 | G07912 | 0.194 | 6 | 0.306 | 0.179 | 6 | 0.602 | 0.248 | 9 | 0.624 | 0.207 | 7 | 0.627 | 0.076 | 9 | 0.284 |
| 16 | D16S00261 | 0.107 | 8 | 0.696 | 0.358 | 9 | 0.762 | 0.138 | 10 | 0.658 | 0.037 | 11 | 0.389 | 0.047 | 11 | 0.443 |
| 17 | D17S00441 | 0.104 | 16 | 0.888 | 0.361 | 15 | 0.999 | 0.061 | 12 | 0.415 | 0.064 | 23 | 0.706 | 0.053 | 24 | 0.590 |
| 18 | D18S00131 | 0.123 | 9 | 0.299 | 0.499 | 8 | 0.933 | 0.172 | 8 | 0.280 | 0.315 | 10 | 0.961 | 0.382 | 10 | 0.920 |
| 19 | D19S00191 | 0.498 | 8 | 0.977 | 0.216 | 7 | 0.564 | 0.078 | 8 | 0.385 | 0.036 | 8 | 0.183 | 0.290 | 8 | 0.581 |
| 20 | D20S00301 | 0.247 | 7 | 0.557 | 0.047 | 7 | 0.149 | 0.339 | 7 | 0.724 | 0.503 | 8 | 0.970 | 0.125 | 9 | 0.534 |
| 21 | D21S00361 | 0.036 | 13 | 0.558 | 0.109 | 11 | 0.339 | 0.050 | 11 | 0.099 | 0.060 | 13 | 0.365 | 0.059 | 15 | 0.626 |
| 22 | D22S01551 | 0.246 | 4 | 0.443 | 0.003 | 4 | 0.010 | 0.124 | 4 | 0.207 | 8.07E-05 | 4 | 3.44E-04 | 0.001 | 4 | 0.010 |
| X | HUMUT1223 | 0.115 | 5 | 0.515 | 0.140 | 6 | 0.416 | 0.122 | 5 | 0.182 | 0.006 | 6 | 0.069 | 0.021 | 6 | 0.081 |
| Pritchard's chi square df **P value** | | | 69.4 | | | 81.8 | | | 85.4 | | | 70.0 | | | 54.6 | |
| | | | 65 | | | 64 | | | 62 | | | 65 | | | 65 | |
| | | | **0.331** | | | **0.066** | | | **0.361** | | | **0.314** | | | **0.818** | |

After the population stratification test, three pooled DNA templates from each case or control population were used in three-phased genomic screening. This screening method simply means reproduction in three independent sample populations and is known to be suitable for excluding many false positives due to Type I errors caused by multiple testing (Barcellos, L. et al., Am. J. Hum. Genet., 61, 724 (1997)). The first (first-phase) screening indicated that 2,847 MS markers were statistically significant (P<0.05) by the Fisher's exact test for either 2×2 or 2×m contingency tables (m=the number of alleles). Subsequent second (second-phase) screening indicated that of these 2, 847 markers, 372 MS markers were significant. After further third (third-phase) screening, 133 positive MS markers were obtained. These results are shown in Table 3.

**[Table 3]**

| **Supplementary Table 3. Summary of the phased genome screen by the pooled DNA method** | | | | | | |
|---|---|---|---|---|---|---|
| **Chromosome** | **Screening phases** | | | | | |
| | **1st (n=125 each)** | | **2nd (n=125 each)** | | **3rd (n=125 each)** | |
| | **Number of Marker** | **Number of Positive*** | **Number of Marker** | **Number of Positive** | **Number of Marker** | **Number of Positive** |
| **1** | **2,241** | **232** | **232** | **27** | **25** | **10** |
| **2** | **2,373** | **249** | **249** | **29** | **25** | **7** |
| **3** | **1,991** | **204** | **204** | **33** | **30** | **8** |
| **4** | **1,740** | **184** | **184** | **23** | **23** | **11** |
| **5** | **1,733** | **168** | **168** | **22** | **20** | **11** |
| **6** | **1,619** | **170** | **170** | **36** | **29** | **10** |
| **7** | **1,599** | **201** | **201** | **25** | **23** | **9** |
| **8** | **1,375** | **124** | **124** | **14** | **11** | **4** |
| **9** | **1,101** | **135** | **135** | **9** | **9** | **4** |
| **10** | **1,281** | **127** | **127** | **18** | **16** | **7** |
| **11** | **1,303** | **139** | **139** | **16** | **13** | **5** |
| **12** | **1,260** | **144** | **144** | **16** | **12** | **3** |
| **13** | **893** | **99** | **99** | **12** | **9** | **6** |
| **14** | **762** | **79** | **79** | **19** | **19** | **4** |
| **15** | **689** | **71** | **71** | **9** | **7** | **4** |
| **16** | **732** | **57** | **57** | **12** | **13** | **5** |
| **17** | **725** | **77** | **77** | **6** | **4** | **2** |
| **18** | **750** | **86** | **86** | **8** | **7** | **5** |
| **19** | **503** | **67** | **67** | **10** | **11** | **3** |
| **20** | **565** | **50** | **50** | **7** | **7** | **2** |
| **21** | **324** | **37** | **37** | **4** | **4** | **1** |
| **22** | **293** | **33** | **33** | **6** | **5** | **4** |
| **X** | **1,187** | **114** | **114** | **11** | **13** | **8** |
| **Total** | **27,039** | **2847 (1,377)** | **2,847** | **372 (215)** | **335** | **133 (53)** |

| | | | | | | |
|---|---|---|---|---|---|---|
| *Number of positive markers by the Fisher exact test for the 2x2 or 2xm contingency tables. The number of positive markers by 2xm is indicated in parenthesis. | | | | | | |

The number of the positive MS markers was larger than statistically expected, suggesting that experiment errors caused by the pooled DNA method were contained therein, as previously reported (Shaw, S.H. et al., Nat. Rev. Genet., 3, 862 (2002)). Thus, we carefully verified these positive markers by individual genotyping in the screened populations. As a result, 47 markers were significant. of these markers, 25 were excluded due to their low positive allele frequencies (<0.05), resulting in a list of 23 positive Ms markers (Table 4)

### [Table 4]

**Table 1. Twenty-fhre positive microsatellite markers from individual genotyping**

| **Markers** | **Number of Positive** | | | **Allele frequencies** | | **Fishers exact P values** | | | | **Odds Ratio** | **95%Cl** |
|---|---|---|---|---|---|---|---|---|---|---|---|
| | **Cvtobands** | **allele** | **allele** | **Control** | **Case** | **2x2** | **PC** | **2xm** | **PC** | | |
| **D6SO5881** | **6p21.3** | **10** | **5** | **0.430** | **0.572** | **0.000000055** | **0.000014** | **0** | **0** | **1.78** | **1.45 - 2.18** |
| **D6S04831** | **6p21.3** | **18** | **7** | **0.089** | **0.176** | **0.00000092** | **0.00024** | **0** | **0** | **2.18** | **1.59 - 2.98** |
| **D6S1061** | **6p21.3** | **24** | **16** | **0.095** | **0.183** | **0.000001** | **0.00026** | **0** | **0** | **2.14** | **1.57 - 2.90** |
| **DIIS04971** | **11q13.4** | **5** | **2** | **0.513** | **0.613** | **0.000031** | **0.008** | **0.00052** | **0.012** | **1.55** | **1.26 - 1.91** |
| **D6SO025i** | **6p21.3** | **6** | **2** | **0.125** | **0.185** | **0.002** | **0.51** | **0.0005** | **0.012** | **1.59** | **1.20 - 2.11** |
| **D10S0168i** | **10p13** | **4** | **2** | **0.408** | **0.499** | **0.0005** | **0.13** | **0.001** | **0.024** | **1.44** | **1.18 - 1.77** |
| **D14SO452i** | **14q23.1** | **9** | **4** | **0.370** | **0.452** | **0.001** | **0.26** | **0.0006** | **0.014** | **1.40** | **1.14 - 1.72** |
| **D8S0127i** | **8q13.3** | **16** | **3** | **0.116** | **0.069** | **0.002** | **1** | **0.009** | **0.25** | **0.57** | **0.40 - 0.81** |
| **D7S0086i** | **7p21.1** | **11** | **4** | **0.095** | **0.053** | **0.002** | **1** | **0.03** | **0.75** | **0.54** | **0.36 - 0.80** |
| **D10S0607i** | **10q26.13** | **5** | **1** | **0.827** | **0.882** | **0.003** | **1** | **0.02** | **0.5** | **1.59** | **1.19 - 2.14** |
| **D13S0561i** | **13q31.1** | **10** | **8** | **0.130** | **0.183** | **0.005** | **1** | **0.16** | **1** | **1.50** | **1.13 - 2.00** |
| **G08462** | **5q14.1** | **9** | **4** | **0.190** | **0.136** | **0.005** | **1** | **0.09** | **1** | **0.67** | **0.51 - 0.89** |
| **D16S0496i** | **16q12.2** | **10** | **7** | **0.204** | **0.267** | **0.005** | **1** | **0.07** | **1** | **1.41** | **1.11 - 1.79** |
| **D5S0228i** | **5q12.1** | **11** | **7** | **0.305** | **0.371** | **0.004** | **1** | **0.02** | **0.5** | **1.35** | **1.09 - 1.67** |
| **D5S400** | **5q34** | **18** | **2** | **0.063** | **0.101** | **0.008** | **1** | **0.03** | **0.75** | **1.69** | **1.15 - 2.46** |
| **D6S0811i** | **6q22.33** | **6** | **3** | **0.445** | **0.515** | **0.008** | **1** | **0.01** | **0.25** | **1.31** | **1.07 - 1.61** |
| **D20S910** | **20p12.1** | **14** | **7** | **0.301** | **0.365** | **0.009** | **1** | **0.18** | **1** | **1.34** | **1.08 - 1.66** |
| **D4S0017i** | **4q25** | **22** | **5** | **0.071** | **0.111** | **0.009** | **1** | **0.12** | **1** | **1.64** | **1.14 - 2.35** |
| **D16S0232i** | **16q24.1** | **4** | **2** | **0.444** | **0.380** | **0.01** | **1** | **0.06** | **1** | **0.77** | **0.63 - 0.95** |
| **D3S1500i** | **3p24.3** | **4** | **1** | **0.781** | **0.725** | **0.01** | **1** | **0.005** | **0.13** | **0.74** | **0.58 - 0.94** |
| **D20S470** | **20p12.1** | **14** | **7** | **0.111** | **0.073** | **0.02** | **1** | **0.59** | **1** | **0.64** | **0.45 - 0.91** |
| **DXS0486i** | **Xq25** | **8** | **1** | **0.118** | **0.090** | **0.09** | **1** | **0.19** | **1** | **0.68** | **0.51 - 1.04** |
| **D18S0090i** | **18q12.1** | **20** | **13** | **0.193** | **0.153** | **0.05** | **1** | **0.54** | **1** | **0.76** | **0.58 - 0.99** |

| | | | | | | | | | | | |
|---|---|---|---|---|---|---|---|---|---|---|---|
| PC means corrected P values by Bonferroni's correction. The Fisher's exact test was earned out in the case and control populations (n=375 each). This means allele frequency of which has the lowest P value in the locus. | | | | | | | | | | | |

Specific data serving as a basis for Table 4 are shown in Table 5. As an example, this table classifies the region determined by each MS marker as positive (+) (which was judged as having significant disease association in the rheumatoid arthritis group (P) as compared with the normal individual group (C)) or as negative (-). For example, "+/+" means that both alleles are positive, and "+/-" means that one of alleles is positive, according to the classification. The use of this table allows for, for example, the digitization of the possibility of rheumatoid arthritis onset by grading each test subject according to specific algorithm on the basis of these numeric values. In the table, "o" denotes mistyping.

**[Table 5]**

| | | **D6S0588i** | | **D6S1061** | | **D6S0483i** | | **D6S0025i** | | **D11S0497i** | | **D10S0168i** | | **D14S0452i** | | | |
|---|---|---|---|---|---|---|---|---|---|---|---|---|---|---|---|---|---|
| | | **C** | **P** | **C** | **P** | **C** | **P** | **C** | **P** | **C** | **P** | **C** | **P** | **C** | **P** | | |
| **-** | **-** | **274** | **192** | **311** | **254** | **314** | **255** | **317** | **290** | **219** | **158** | **299** | **287** | **350** | **311** | | |
| **+** | **+** | **199** | **316** | **5** | **11** | **14** | **18** | **37** | **54** | **277** | **340** | **190** | **202** | **155** | **174** | | |
| **+** | **-** | **450** | **421** | **57** | **110** | **43** | **101** | **20** | **31** | **428** | **435** | **446** | **450** | **409** | **448** | | |
| **o** | **o** | **15** | **52** | **565** | **606** | **567** | **607** | **564** | **606** | **14** | **48** | **3** | **42** | **24** | **48** | | |
| **Total** | | **938** | **981** | **938** | **981** | **938** | **981** | **938** | **981** | **938** | **981** | **938** | **981** | **938** | **981** | | |
| | | | | | | | | | | | | | | | | | |

| | | **D8S0127i** | | **D7S0086i** | | **D10S0607i** | | **D1350561** | | **G08482** | | **D16S0496i** | | **D5SO228i** | | **D5S400** | |
|---|---|---|---|---|---|---|---|---|---|---|---|---|---|---|---|---|---|
| | | **C** | **P** | **C** | **P** | **C** | **P** | **C** | **P** | **C** | **P** | **C** | **P** | **C** | **P** | **C** | **P** |
| **-** | **-** | **727** | **763** | **793** | **798** | **20** | **16** | **650** | **647** | **617** | **662** | **568** | **514** | **189** | **152** | **789** | **754** |
| **+** | **+** | **17** | **12** | **8** | **5** | **672** | **692** | **42** | **54** | **40** | **18** | **61** | **61** | **43** | **55** | **8** | **7** |
| **+** | **-** | **163** | **155** | **125** | **117** | **238** | **227** | **215** | **220** | **272** | **245** | **292** | **355** | **142** | **168** | **130** | **175** |
| **o** | **o** | **31** | **9** | **12** | **19** | **8** | **4** | **31** | **18** | **9** | **14** | **17** | **9** | **564** | **564** | **11** | **3** |
| **Total** | | **938** | **939** | **938** | **939** | **938** | **939** | **938** | **939** | **938** | **939** | **938** | **939** | **938** | **939** | **938** | **939** |
| | | | | | | | | | | | | | | | | | |

| | | **D6S0811i** | | **D20S910** | | **D4S0017i** | | **D16S0232** | | **D3S1500i** | | **D20S470** | | **D18S0090i** | | **DXS0486i** | |
|---|---|---|---|---|---|---|---|---|---|---|---|---|---|---|---|---|---|
| | | **C** | **P** | **C** | **P** | **C** | **P** | **C** | **P** | **C** | **P** | **C** | **P** | **C** | **P** | **C** | **P** |
| **-** | **-** | **270** | **250** | **215** | **194** | **324** | **301** | **286** | **352** | **68** | **64** | **296** | **324** | **244** | **268** | **296** | **318** |
| **+** | **+** | **209** | **241** | **65** | **93** | **3** | **9** | **170** | **152** | **528** | **471** | **5** | **4** | **13** | **8** | **13** | **7** |
| **+ -** | **6** | **449** | **437** | **93** | **88** | **47** | **65** | **479** | **430** | **318** | **351** | **73** | **47** | **117** | **99** | **63** | **50** |
| **o** | **o** | **10** | **11** | **565** | **564** | **564** | **564** | **3** | **5** | **24** | **53** | **564** | **564** | **564** | **564** | **566** | **564** |
| **Total** | | **938** | **939** | **938** | **939** | **938** | **939** | **938** | **939** | **938** | **939** | **938** | **939** | **938** | **939** | **938** | **939** |

The seven most significant markers in the list of Table 4 were also significant after Bonferroni's correction (Pc<0.05). Therefore, in this Example, SNP genotyping was focused on these candidate regions.

### SNP Genotyping:

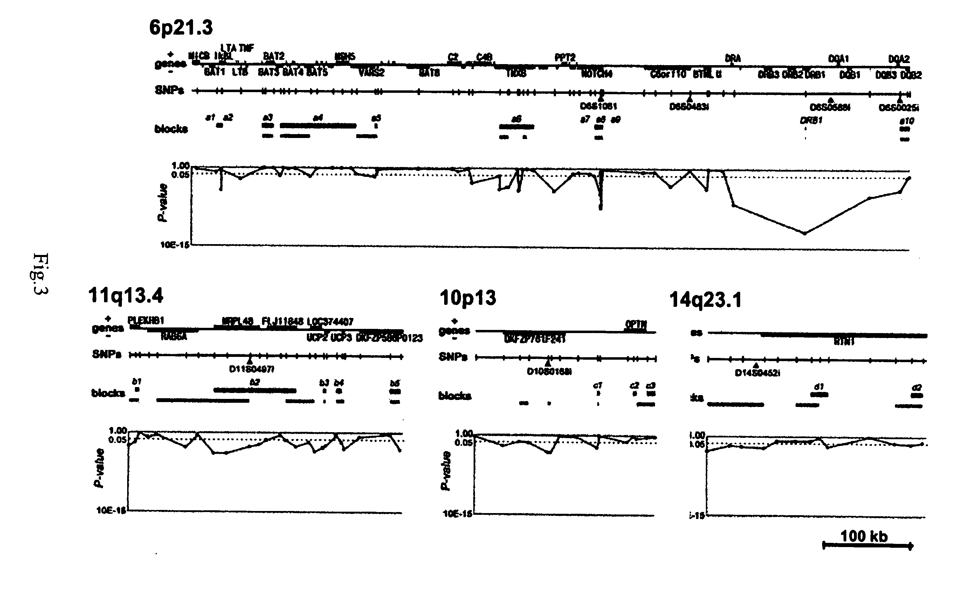

Among the seven most significant markers, four (i.e., the first, second, third, and fifth) were located in the HLA region on 6p21.3 (Figure 3), whereas the fourth, sixth, and seventh significant markers were located on 11q13.4, 10p13, and 14q23.1, respectively (cytobands are designated under the NCBI build 30).

SNPs in the neighborhoods of these candidate regions were selected from dbSNP database of NCBI homepage and JSNP database of the homepage of The Institute of Medical Science, The University of Tokyo. These SNPs were genotyped using TaqMan assay or direct sequencing. The TaqMan assay was conducted using the standard protocol of ABI PRISM 7900HT Sequence Detection System (Applied Biosystems) equipped with 384-Well Block Module and Automation Accessory. The direct sequencing of the PCR products was conducted according to a standard approach using ABI3700 DNA analyzer (Applied Biosystems). In the HLA region, additional SNPs were selected from IkBL to C4B genes in order to verify previously reported RA association around the centromeric end of the HLA class III region. See Table 6 for the details of the selected SNPs.

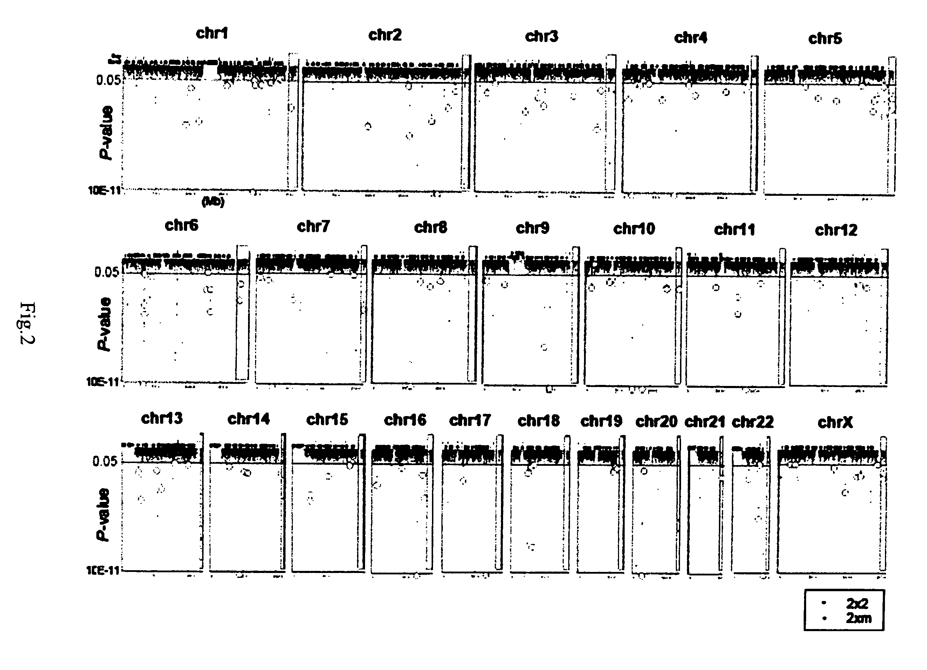

Genotyping was conducted on 165 SNPs in the case and control populations used in the MS typing. Of these SNPs, 41 were neither polymorphic nor STSs (sequence tagged sites) (see Table 6) and were therefore excluded from subsequent analysis. Among the remaining 124 SNPs, 54 were statistically significant by case-control association analysis (P<0.05) (Table 7). LD block structures were predicted for these 124 SNPs by EM algorithm (Figure 2), and case-control association analysis usinghaplotypes in eachblockwas conducted according to this algorithm (Table 8). To reproduce these SNP allelic associations, these 54 positive SNPs were genotyped in additional populations composed of 565 case individuals and 565 control individuals. Finally, 45 positive SNPs were obtained in the combined (n=2x940) population consisting of all the samples used in this experiment. Among these positive SNPs, 24 was also significant (Pc<0.05) after Bonferroni's correction (Table 7).

Hereinafter, the analysis result of each chromosome will be described.

### 6p21.3

In the HLA region on 6p21.3, 28 of 71 polymorphic SNPs were statistically significant (Pc<0.05) in the first test. Preliminary genotyping on HLA-DRB1 revealed that the HLA-DRB1*0405 allele was most significant (P=1.3×10⁻¹²). The result was, as expected, consistent with many previous reports on Japanese populations (Wakitani, S. et al., Br. J. Rheumatol., 36, 630 (1997); and Shibue, T. et al, Arthritis Rheum., 43, 753 (2000)) and demonstrated that the method used in the present invention is effective for detecting the association of susceptibility genes with RA. In addition to HLA-DRB1, the association of the IkBL gene (MIM*601022) promoter SNP rs3219185 was also reproduced (P=5.4' 10⁻⁵), albeit with relatively low frequency of the minor allele. Moreover, strong association was seen around the NOTCH4 (MIM*164951) and TNXB (MIM*600985) genes, which were approximately 250 kb and 300 kb, respectively, distant from HLA-DRB1.

The NOCTH4 gene is one of proto-oncogenes with epidermal growth factor (EGF) repeats. NOTCH4 encodes a large transmembrane receptor predicted to be involved in the signal transduction of cell proliferation, cell differentiation, and angiogenesis (Yung Yu, C. et al., Immunol. Today, 21, 320 (2000)). In NOTCH4, nine SNPs were statistically significant, among which two caused amino acid exchange. Among these nine SNPs, rs2071282, the SNP in exon 4, exhibited the strongest association (P=3.1×10⁻⁸) and caused Leu203Pro exchange at the fourth EGF repeat in the extracellular domain of NOTCH4. On the other hand, rs915894 in exon 3 was moderately significant (P=0.044) and caused Lys116Gln exchange at the third EGF repeat.

The TNXB gene encodes one of extracellular matrix proteins with 34 fibronectin type III-like (FNIII) and 18 EGF repeats and participates in at least one of essential functions of collagen deposition in connective tissues (Mao, J. R. et al., Nat. Genet. 30, 421 (2002)). In TNXB, five SNPs were statistically significant, of which four caused amino acid exchange. Among these five SNPs, rs185819 in exon 10 exhibited the strongest association (P=6.8×10⁻⁵) and caused His1248Arg exchange at the seventh FNIII repeat. Other SNPs, rs2075563 (Glu3260Lys) in exon 29, rs2269428 (His2363Pro) in exon 21, and rs3749960 (Phe2300Tyr) in exon 20, were also significant and located in the 26th, 18th, and 17th FNIII repeats, respectively.

These six positive SNPs were finally confirmed in the combined (n=2x940) population (Table 6). Further, haplotype analysis demonstrated these results for IkBL, NOTCH4, and TNXB (Table 7), indicating the absence of, in all blocks of each gene, common haplotypes with greater risks than that of single SNP in each gene. When multiple logistic regression analysis was conducted for the SNPs in IkBL, TNXB, and NOTCH4 with those in HLA-DRB1, three genes, DRB1*0405 (ORs=2.29-8.84), rs3219185 in IkBL (ORs=1.16-2.67), and rs185819 in TNXB (ORs=1.00-1.62), were significant (P<0.05) in a partially recessive model. Two SNPs, DRB1 (ORs=2.16-4.69) and TNXB (ORs=1.02-1.84), were significant in a partially dominant model. On the other hand, when the analysis was limited to the shared epitope (SE) of DRB1, SE (ORs=1.79-3.85), IkBL (ORs=1.11-2.54), and rs2071282in NOTCH4 (ORs=1.13-7.14) were significant only in the partially recessive model. These results suggested that these loci independently correspond to RA in the partially recessive model. 11q13.4

The candidate region on 11q13.4 contained nine genes includingthreemitochondrial-relatedgenesMRPL48, UCP2, and UCP3. Although MRPL48 was recently found as a gene having homology to mammalian mitochondrial ribosomal proteins (MRPs) (Zhang, Z. and Gerstein, M., Genomics, 81, 468 (2003)), its function is still unknown. UCP2 (MIM*601693) and UCP3 (MIM*602044) encode transporter proteins on the inner mitochondrial membrane and participate in energy consumption. UCP2 is also known as a susceptibility gene for obesity and diabetes. RAS-associated protein RAB6A (MIM*179513) was centromerically found with respect to MRPL48. Further, three novel genes were located in regions FLJ11848, LOC374407, and DKFZP586P0123. FLJ11848 has WD40 repeats and widely participates in cell-cell interaction (Smith, T. F. et al., Trends Biochem. Sci., 24, 181 (1999)). LOC374407 has been found to have homology to heat shock protein 40 homolog (HSP40 homolog) and structural similarity to spermatogenesis apoptosis-related protein. DKFZP586P0123 has one protein kinase C conserved region.

In these genes, 16 of 25 polymorphic SNPs were statistically significant in the first test. Although these positive SNPs were scattered over the region tested, most significant associations (P=0.00015) were observed in two SNPs, rs1792174 in 5' -UTR and rs1792160 in intron 3 of MRPL48. MRPL48 also had two other positive SNPs, rs1792193 (P=0.003) inintron 5 and rs1051090 (P=0.007) in 3'-UTR. Positive SNPs were also observed in all of other genes UCP2, UCP3, RAB38 and FLJ11848. However, only one common haplotype in the block b2 containing MRPL48 and FLJ11848 exhibited significant association as strong as the single SNP in MRPL48. These positive SNPs in MRPL48 were finally confirmed after Bonferroni's correction in the combined population (Table 7). On the other hand, rs1527302 in DKFZP586P0123 was significant (P=0.00078) both in the first test and after haplotype analysis. However, the SNP allelic association was not confirmed in the combined population. These results suggested that other causative SNPs are present in the block b2.

### 10q13, 14q23.1, and PADI4

The candidate region on 10p13 had two genes, DKFZP761F241 and optineurin (OPTN). Three SNPs in the DKFZP761F241 gene were statistically significant in the first test and however, was not confirmed after correction in the combined population. No common haplotype existed in regions that remained after Bonferroni's correction in each population.

On the other hand, the candidate region on 14q23.1 contained only reticulon 1 gene (MIM*600865), which encodes the neuroendocrine-specific protein group. Even after Bonferroni's correction in the combined samples, rs2182138 in intron 3 of RTN1 was still statistically significant (P=0.0002). No common haplotype was observed in both regions that remained after correction.

Further, in the PADI4 gene that appeared to be a susceptibility gene for RA by the candidate gene approach (Non-Patent Document 5), four positive SNPs, padi89 (P=0.002), padi90 (P=0.004), rs874881(P=0.002), and rs2240340 (P=0.002), were replicated in the populations of this Example. D1S1144i, a CA microsatellite marker in intron 6 of the PADI4 gene, was confirmed to be included in the RAmarker set and exhibit slight significance (P=0.008) but low associated allele frequency (P=0.037 in the control population).

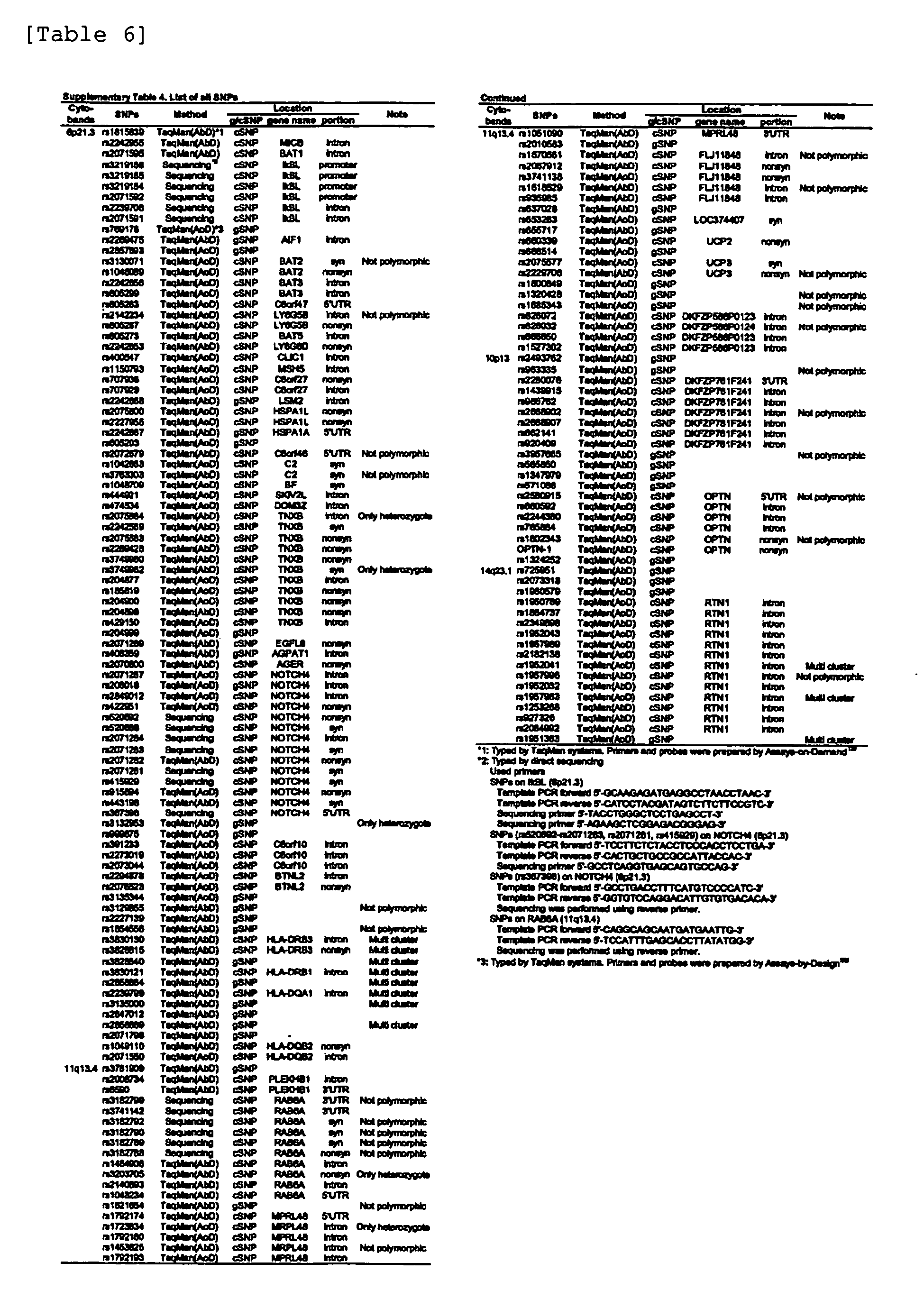

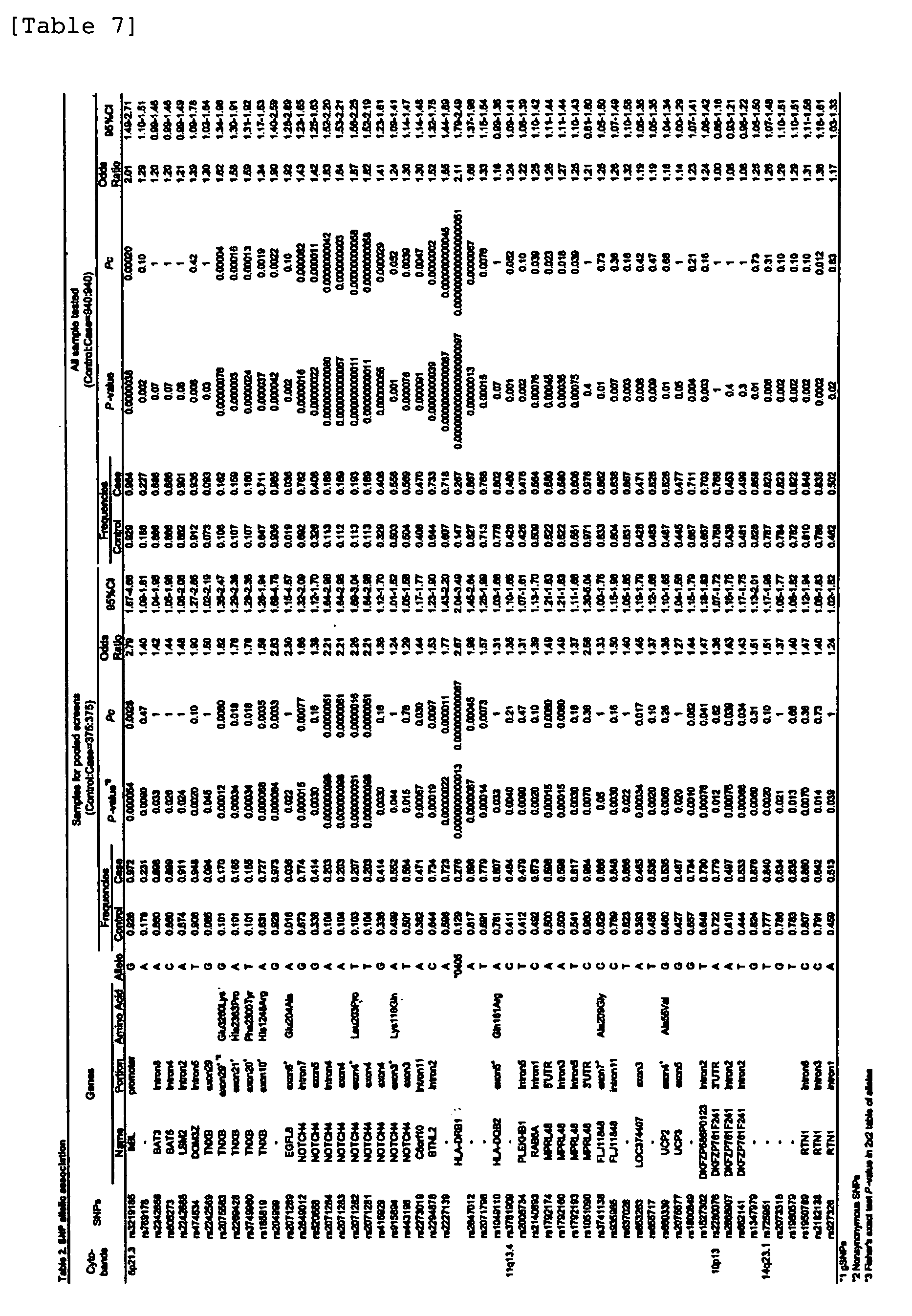

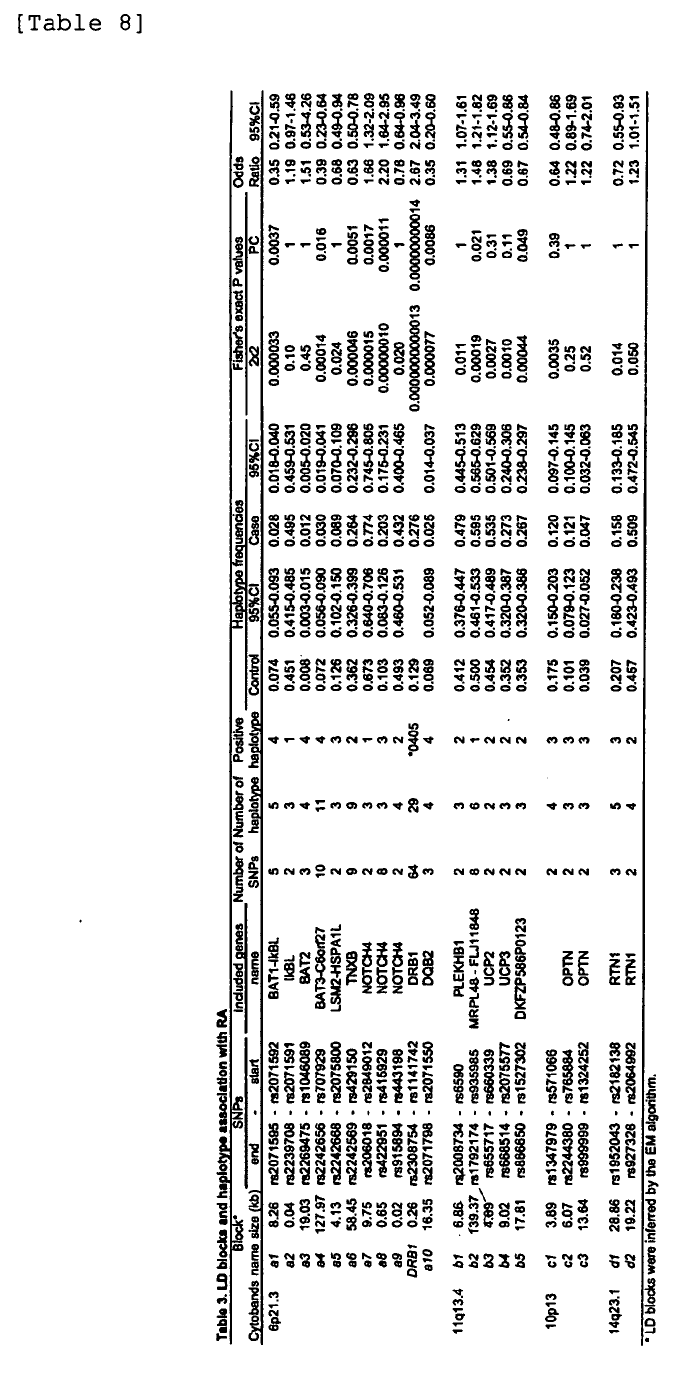

### Expression Analysis

To study the expression patterns of these genes in various tissues including synovial cells, we performed quantitative reverse transcription-PCR (QRT-PCR) using RNA from these tissues.

Total RNA was isolated by ISOGEN (Nippon Gene) from synovial membranes surgically obtained from eight RA and four osteoarthritis (OA) patients. We also isolated total RNA from a synovial cell line (SW982) provided by American Type Culture Collection (ATCC). Other RNAs from various tissues are commercially available from Clontech, Invitrogen, Origene, and Stratagene. We evaluated the quality and quantity of these RNAs by use of Agilent 2100 Bioanalyzer (Agilent) and confirmed their quantities by RiboGreen RNA fluorescence assay (Molecular Probes). Complimentary DNAs were synthesized from these total RNAs using random hexamers and TaqMan reverse transcription reagents kit (Applied Biosystems). We obtained cDNA-specific primers and probes by the 'Assay-by-Design (AbD)' for the ten genes tested and by the 'Assay-on-Demand (AoD) ' for GAPD used as a housekeeping control gene, all of which were provided by Applied Biosystems. After preliminary experiments, 210 nM probes, 756 nM primers, and 0.48 ng/ml cDNA at the final concentration in 50 ml reaction volume were used in 96-well reaction plates on ABI PRISM 7900 according to the standard approach recommended by Applied Biosystems. Each plate was processed three times to calculate the average and SD for each sample. Estimated quantity was calculated each time using a standard curve in each well. All quantity data normalized to GAPD were tested by the Smirnov's test with a 5% significance level. After the reciprocal transformation of all the normalized quantity data, the Student's t-test was conducted for expression levels between RA and OA synovial tissues.

The consistently high expression of NOTCH4 in the lung and of TNBX in the adrenal gland were observed (Figure 3a) . Our results also showed that all the genes were expressed in the RA synovial cells. TNXB and NOTCH4 had significantly high expression levels in the RA synovial cells, whereas RTN1 had the lowest level. We also compared the expression levels of these genes between RA and OA synovial cells. The expression levelsoftheMRPL48 (P=0.049) and DKFZP761F241 (P=0.027) genes exhibited relatively significant difference between the RA and OA synovial cells by the Student's t-test (Table 9 and Figure 3b). MRPL48 expression in the RA synovial tissue was approximately twice that in the OA tissue. Three-quarters of the RA tissue donors were homozygous for a positive haplotype in the block b2 of the MRPL48 locus.

**[Table 9]**

| **Table 4. Expression levels of RA candidate genes in OA and RA synovial cells** | | | | |
|---|---|---|---|---|
| Gene | OA synovial cell | | RA synovial cell | |
| | Average | S.D. | Average | S.D. |
| IkBL | 3.1 | 2.4 | 1.6 | 0.8 |
| TNXB | 339.3 | 349.7 | 80.7 | 35.1 |
| NOTCH4 | 36.7 | 0.4 | 39.7 | 30.8 |
| MRPL48 | 2.5* | 0.2 | 4.4 | 1.6 |
| FLJ11848 | 0.9 | 0 | 1.3 | 0.7 |
| UCP2 | 1.5 | 0.9 | 2.8 | 2.1 |
| DKFZP761 F241 | 17.5* | 8.1 | 7.1 | 0.8 |
| OPTN | 7.4 | 2.4 | 12.7 | 3.4 |
| RTN1 | 0.8 | 0.1 | 0.9 | 0.6 |
| BLT2** | 51.2 | 70.7 | 11.1 | 6.9 |

| | | | | |
|---|---|---|---|---|
| * Expression levels of MRPL48 (P=0.049) and DKFZP761F241 (P=0.027) genes showed relatively significant difference between RA and OA synovial tissues. ** The BLT2 (leukotriene B4 receptor subtype 2) gene was employed as a positive control, which has been known to have strong expression in the RA | | | | |

### Statistical Analysis:

To calculate P-values, two types of the Fisher's exact test were used for the 2×2 contingency tables for each individual allele and the 2×m contingency tables for each locus. In this context, m refers to the number of markers observed in a population. To practice the Fisher's exact test for the 2×m contingency tables, Markov chain/Monte Carlo simulation method was adopted. We simply presented "allelic" but not phenotypic association for the 2x2 contingency tables for MS, SNP and haplotype. These P-values were corrected by Bonferroni's correction, wherein the coefficient was the total number of the contingency tables tested. These analyses were conducted with software package MCFishman. Other basic statistical analyses including multiple logistic regression analysis and Mantel-Haenszel test were performed using SPSS program package and Microsoft Excel (trade name). We predicted LD block structures for these SNPs by using the confidence intervals of the D' value as a LD measure (Gabriel, S. B. et al., Science, 296, 2225 (2002); and Dawson, E. et al., Nature, 418, 544(2002)). Moreover, haplotypes in each block and their frequencies were estimated by EM and Clark algorithms. Finally, to evaluate the reliability of haplotypes in each block, the 95% confidence interval was calculated from each haplotype frequency given by bootstrap resampling of up to 2000 times on the basis of the estimated haplotype frequencies, which was implemented in the Right program (Mano. S. et al., Ann. Hum. Genet., in press).

In this Example, strong association was found in TNXB and NOCTH4 genes 250 kb distant from HLA-DRB1 in the candidate region narrowed down by the MS markers. These genes are known to be located in LD blocks evidently different from that of HLD-DRB1 (Cullen, M. et al., Am. J. Hum. Genet., 71, 759 (2002) ; and Walsh, E. C. et al., Am. J. Hum. Genet., 73, 580 (2003)). In agreement with the multiple logistic regression analysis result, the result of Mantel-Haenszel test also showed that positive SNPs in TNXB and NOTCH4 are independent of HLA-DRB1*0405 or SE in both partially dominant and partially recessive models (data not shown). Further, the candidate region was highly identical to one of additional susceptibility regions previously predicted (Jawaheer, D. et al., Am. J. Hum. Genet., 71, 585 (2002)). TNXB is known as a causative gene of one type of Ehlers-Danlos syndrome (MIM*600985) characterized by dysfunction in connective tissues including joints. Its gene products participate in connective tissue functions and in structures via the deposition of collagens of various types (Mao, J. R. et al., Nat. Genet., 30, 421 (2002)), probably including synovial tissues shown here. Type II collagen-induced arthritis inmice is known tomimic rheumatoid arthritis (Moore. AR., Methods Mol. Biol., 225, 175 (2003)).

The present inventors believe that the amino acid exchanges of the TNXB gene product serve as functional factors for RA via a hypothetical pathway associated with collagen metabolism. In recent years, it was reported that the NOTCH4 gene product might participate in overproliferation via tumor necrosis factor (TNF) of synovial cells and in RA (Ando, K. et al., Oncogene, 22, 7796 (2003)). However, large parts of NOTCH4 function are still unclear.

On 11q13.4, although MRPL48 function is still unknown, its expression pattern indicated the association of this gene with RA. The candidate region 11q13.4 contains other interesting genes RAB6A, FLJ11848, UCP2, and UCP3. As with this region, even though further association analysis for 10q13 and 14q23.1 requires using higher-density SNP markers, it is interesting that other chromosomes were found by the method of the present invention. These results chiefly suggested that our marker set and method are highly practicable and applicable to other complicated diseases, at least to oligogene diseases with major genes such as HLA-DRB1 in RA.

Interestingly, our data suggested that the seven most significant MS markers are individually positioned in particular LD blocks as a trend (Figure 3). These markers were observed on the "Clarkblocks" rather than the "EMblocks". In many cases, positive MS alleles were obviously associated with positive SNP haplotypes in these blocks.

## Claims

1. A marker gene for rheumatoid arthritis test consisting of a consecutive partial DNA sequence comprising at least one base exhibiting single nucleotide polymorphism present in a TNXB, NOTCH4, RAB6A, MPRL48, UCP2 or UCP3 gene in the human genomic DNA sequence, or of a complementary strand of the partial DNA sequence.

2. The marker gene according to claim 1, wherein the base exhibiting single nucleotide polymorphism is **characterized by** being selected from the group consisting of:
the 61st base in SEQ ID NO: 1 or a corresponding base on a complementary strand thereof;
the 61st base in SEQ ID NO: 2 or a corresponding base on a complementary strand thereof;
the 61st base in SEQ ID NO: 3 or a corresponding base on a complementary strand thereof;
the 61st base in SEQ ID NO: 4 or a corresponding base on a complementary strand thereof;
the 401st base in SEQ ID NO: 5 or a corresponding base on a complementary strand thereof;
the 495th base in SEQ ID NO: 6 or a corresponding base on a complementary strand thereof;
the 61st base in SEQ ID NO: 7 or a corresponding base on a complementary strand thereof;
the 61st base in SEQ ID NO: 8 or a corresponding base on a complementary strand thereof;
the 61st base in SEQ ID NO: 9 or a corresponding base on a complementary strand thereof;
the 61st base in SEQ ID NO: 10 or a corresponding base on a complementary strand thereof;
the 401st base in SEQ ID NO: 11 or a corresponding base on a complementary strand thereof;
the 401st base in SEQ ID NO: 12 or a corresponding base on a complementary strand thereof;
the 401st base in SEQ ID NO: 13 or a corresponding base on a complementary strand thereof;
the 503rd base in SEQ ID NO: 14 or a corresponding base on a complementary strand thereof;
the 201st base in SEQ ID NO: 15 or a corresponding base on a complementary strand thereof;
the 511th base in SEQ ID NO: 16 or a corresponding base on a complementary strand thereof;
the 201st base in SEQ ID NO: 17 or a corresponding base on a complementary strand thereof;
the 51st base in SEQ ID NO: 18 or a corresponding base on a complementary strand thereof;
the 61st base in SEQ ID NO: 19 or a corresponding base on a complementary strand thereof;
the 497th base in SEQ ID NO: 20 or a corresponding base on a complementary strand thereof;
the 201st base in SEQ ID NO: 21 or a corresponding base on a complementary strand thereof; and
the 201st base in SEQ ID NO: 22 or a corresponding base on a complementary strand thereof.

3. The marker gene according to claim 1 or 2, wherein the marker gene is 50 to 1500 bp in length.

4. The marker gene according to claim 3, wherein the marker gene is 100 to 1000 bp in length.

5. A method for testing rheumatoid arthritis comprising collecting a partial DNA sequence corresponding to a marker gene according to any one of claims 1 to 4 from a test subject, determining a nucleotide sequence of the partial DNA sequence, and comparing the nucleotide sequence with a corresponding nucleotide sequence obtained from a normal individual.

6. A test kit for rheumatoid arthritis comprising a marker gene according to any one of claims 1 to 4 or a primer thereof.

7. The test kit according to claim 6, wherein the primer has a DNA sequence represented by any of SEQ ID NOs: 23 to 66.

8. A vector comprising a DNA sequence of a marker gene according to any one of claims 1 to 4.

9. A host cell transformed with a vector according to claim 8.

10. A polypeptide encoded by a marker gene according to any one of claims 1 to 4.

11. A method for producing a polypeptide according to claim 10, comprising incubating a host cell according to claim 8 under conditions suitable for expression.

12. A screening method using a polypeptide according to claim 10.

13. An agonist and/or antagonist obtained by a screening method according to claim 12.

14. A diagnostic, preventive, and/or therapeutic drug for rheumatoid arthritis comprising an agonist and/or antagonist according to claim 13.


SEQUENCE LISTING

<110>  INOKO, Hidetoshi, et al.,
<120>  Novel marker genes for the inspection of rheumatoid arthritis
<130>  JP4694GDP
<160>  66
<170>  Patent In version 3.1

<210>  1
<211>  121
<212>  DNA
<213>  Homo sapiens
<220>
<221>  variation
<222>  (61)..(61)
<223>  /gene="TNXB"
       /replace="A"
       /replace="G"
       /db_xref="dbSNP:2242569"
<400>  1
ggacagggac gggcagcccc aggtggtgcg tgtcaggggc gaggagagcg aggtcaccgt     60
rgggggcctg gagcccgggc gcaaatacaa gatgcatctg tacggcctcc acgaggggca    120
g                                                                    121

<210>  2
<211>  121
<212>  DNA
<213>  Homo sapiens
<220>
<221>  variation
<222>  (61)..(61)
<223>  /gene="TNXB"
       /replace="A"
       /replace="G"
       /db_xref="dbSNP:2075563"
<400>  2
tccttcaccg tgcagtacaa ggacagggac gggcagcccc aggtggtgcg tgtcaggggc     60
raggagagcg aggtcaccgt ggggggcctg gagcccgggc gcaaatacaa gatgcatctg    120
t                                                                    121

<210>  3
<211>  121
<212>  DNA
<213>  Homo sapiens
<220>
<221>  variation
<222>  (61)..(61)
<223>  /gene="TNXB"
       /replace="C"
       /replace="A"
       /db_xref="dbSNP:2269428"
<400>  3
ccccaggaaa ggatgaagaa atggccccag cctcgacaga acctcccacc cctgaacccc     60
mcatcaagcc tcgcctggag gagctgaccg tgacagatgc gacccctgac tccctcagcc    120
t                                                                    121

<210>  4
<211>  121
<212>  DNA
<213>  Homo sapiens
<220>
<221>  variation
<222>  (61)..(61)
<223>  /gene="TNXB"
       /replace="A"
       /replace="T"
       /db_xref="dbSNP:3749960"
<400>  4
gtgaccccat cctcgtgtcc cggcacccgc accgccttgg gctgcccatc cccattctta     60
wactggacca agaaatggtc aaactggccc tcggggactg tccaggagag gctgagggag    120
t                                                                    121

<210>  5
<211>  801
<212>  DNA
<213>  Homo sapiens

<220>
<221> variation
<222> (401)..(401)
<223> /gene="TNXB"
      /replace="C"
      /replace="T"
      /db_xref="dbSNP:185819"
<400> 5
agtctggctg cccctcagcc ctggagtggg gccgggaagc tggagtcagc tgtcttgctg 60
ggggacccca gctggttttg ggctgaaggg aagtgtgcat ggggctgaga aggggtcaca 120
tgggggctga ggtggctgct actcaccagt ggtgccatcg gccgtgaggg ggccataccg 180
cttcttgttc gcaattccaa acagagtgaa tctgtacttg tggtcagggt ccagtgagga 240
gacaacaaat gaacgctcgg gcccttccac aggtaccacc tggggccgtc catccctgtc 300
cctgtactgg accatgaagg tgtcaaactg gccctcaggg acagtccagg agaggtgcag 360
tgaatctggg gtagggtctg tcacccacag gtttcccagg yggggtggag tccctggact 420
tgggtcactc tgaggcacta ggaagagtgg gtagagagaa gggagagact taggtccaag 480
gagaatgggg aagccaaatc ccacatagga atgctgtgtg aggctgtgca ggttgttcac 540
tgcacaaaag tgcatttgct gagggagtac agagggactg aaatccagcc agcactctgc 600
ttgccgagct gtgtgccctg gtgaggagtg gtgtccactt taaggaatgg gtgccttctt 660
tcaaacggca tggaagcact gcgtggacta gtgtggctct gcctccaacc acaaaccaga 720
gcagcaggga gcttcagaaa gaggggagcc cagccaggcc ctttcacatc tccatagcca 780
gggaaatctt cccagtacaa c 801

<210> 6
<211> 995
<212> DNA
<213> Homo sapiens
<220>
<221> variation
<222> (495)..(495)
<223> /gene="NOTCH4"
      /replace="A"
      /replace="G"
      /db_xref="dbSNP:520688"
<400> 6
tgctagaaac ggctccctct gtcctctgcc tcaggtgagc agtgccagct tcgggacttc 60
tgttcagcca acccatgtgt taatggaggg gtgtgtctgg ccacgtaccc ccagatccag 120
tgccactgcc caccgggctt cgagggccat gcctgtgaac gtgatgtcaa cgagtgcttc 180
caggacccag gaccctgccc caaaggcacc tcctgccata acaccctggg ctccttccag 240
tgcctctgcc ctgtggggca ggagggtcca cgttgtgagc tgcgggcagg accctgccct 300
cctaggggct gttcgaatgg gggcacctgc cagctgatgc cagagaaaga ctccaccttt 360
cacctctgcc tctgtccccc aggtgtgtcc tcacaggggc tctccggccg cccctctctc 420
tgggcagggc aggatgtctc cgttggagcc tcctcccaca gctgatccat gaccctgtca 480
ggtttcatag gcccrggctg tgaggtgaat ccagacaact gtgtcagcca ccaatgtcag 540
aatgggggca cttgccagga tgggctggac acctacacct gcctctgccc agaaacctgg 600
acaggtgagt tgtttaagcc acatccatga cacccatggc ccagagagtt ggcccctggc 660
ctcccctact catagggctc ccagccttag ccctcgtccc ctccccaacc ccctgcaggc 720
tgggactgct ccgaagatgt ggatgagtgt gaggcccagg gtccccctca ctgcagaaac 780
gggggcacct gccagaactc tgctggtagc tttcactgcg tgtgtgtgag tggctggggg 840
ggcacaagct gtgaggagaa cctggatgac tgtattgctg ccacctgtgc cccgggatcc 900
acctgcattg accgggtggg ctctttctcc tgcctctgcc cacctggacg cacaggtatg 960
gggtagaggg gtatcaggag gtgggaggta gagaa 995

<210> 7
<211> 121
<212> DNA
<213> Homo sapiens
<220>
<221> variation
<222> (61)..(61)
<223> /gene="NOTCH4"
      /replace="A"
      /replace="G"
      /db_xref="dbSNP:2071284"
<400> 7
actccacctt tcacctctgc ctctgtcccc caggtgtgtc ctcacagggg ctctccggcc 60
rcccctctct ctgggcaggg caggatgtct ccgttggagc ctcctcccac agctgatcca 120
t 121

<210> 8
<211> 121
<212> DNA
<213> Homo sapiens
<220>
<221> variation
<222> (61)..(61)

<223>  /gene="NOTCH4"
      /replace="A"
      /replace="G"
      /db_xref="dbSNP:2071283"
<400>  8
caacgagtgc ttccaggacc caggaccctg ccccaaaggc acctcctgcc ataacaccct      60
rggctccttc cagtgcctct gccctgtggg gcaggagggt ccacgttgtg agctgcgggc     120
a                                                                     121

<210>  9
<211>  121
<212>  DNA
<213>  Homo sapiens
<220>
<221>  variation
<222>  (61)..(61)
<223>  /gene="NOTCH4"
      /replace="C"
      /replace="T"
      /db_xref="dbSNP:2071282"
<400>  9
cgggcttcga gggccatgcc tgtgaacgtg atgtcaacga gtgcttccag gacccaggac      60
yctgccccaa aggcacctcc tgccataaca ccctgggctc cttccagtgc ctctgccctg     120
t                                                                     121

<210>  10
<211>  121
<212>  DNA
<213>  Homo sapiens
<220>
<221>  variation
<222>  (61)..(61)
<223>  /gene="NOTCH4"
      /replace="C"
      /replace="T"
      /db_xref="dbSNP:2071281"
<400>  10
taatggaggg gtgtgtctgg ccacataccc ccagatccag tgccactgcc caccgggctt      60
ygagggccat gcctgtgaac gtgatgtcaa cgagtgcttc caggacccag gaccctgccc     120
c                                                                     121

<210>  11
<211>  801
<212>  DNA
<213>  Homo sapiens
<220>
<221>  variation
<222>  (401)..(401)
<223>  /gene="NOTCH4"
      /replace="A"
      /replace="G"
      /db_xref="dbSNP:415929"
<400>  11
cagcatggtg ccacctgagt ctcctgcctg cagacgtggg gtgcttttca cctcccccaa      60
gatcacccac gtcccagatt ttctcaggca aggccaattt gcaatactct catcatcact     120
ttagaagata tggtcactcc agataaaccc tcccaagcca tgacatcgct cagagcaggg     180
gtgatggaac agagcaaaga aagtatggta ataaagggaa ggaaatatga aaatgagacc     240
cagagataat ccagagtgag cactgggtaa cctcagatgg gctagaattc gtacaatgct     300
agaaacggct ccctctgtcc tctgcctcag gtgagcagtg ccagcttcgg gacttctgtt     360
cagccaaccc atgtgttaat ggaggggtgt gtctggccac rtacccccag atccagtgcc     420
actgcccacc gggcttcgag ggccatgcct gtgaacgtga tgtcaacgag tgcttccagg     480
acccaggacc ctgccccaaa ggcacctcct gccataacac cctgggctcc ttccagtgcc     540
tctgccctgt ggggcaggag ggtccacgtt gtgagctgcg ggcaggaccc tgccctccta     600
ggggctgttc gaatgggggc acctgccagc tgatgccaga gaaagactcc acctttcacc     660
tctgcctctg tcccccaggt gtgtcctcac aggggctctc cggccgcccc tctctctggg     720
cagggcagga tgtctccgtt ggagcctcct cccacagctg atccatgacc ctgtcaggtt     780
tcataggccc gggctgtgag g                                               801

<210>  12
<211>  801
<212>  DNA
<213>  Homo sapiens
<220>
<221>  variation
<222>  (401)..(401)
<223>  /gene="NOTCH4"

/replace="A"
      /replace="C"
      /db_xref="dbSNP:915894"
<400>  12
gacctgccag tgagtgtgcc ttgcaggagt gggagactgg agagaaaggg ggagggagag      60
caggggggga gaggtgagga agtgagacca aagaagaaag agaggaagtg aaggagatga     120
agggaaacaa atgaaggcag aggagggagt gggcaagaat aggaagaggg accagtgatg     180
tgagttttcc tctcctcccc tgcccaggtg tgcccctggc ttcctgggtg agacgtgcca     240
gtttcctgac ccctgccaga acgcccagct ctgccaaaat ggaggcagct gccaagccct     300
gcttcccgct cccctagggc tccccagctc tccctctcca ttgacaccca gcttcttgtg     360
cacttgcctc cctggcttca ctggtgagag atgccaggcc magcttgaag acccttgtcc     420
tccctccttc tgttccaaaa ggggccgctg ccacatccag gcctcgggcc gcccacagtg     480
ctcctgcatg cctggatgga caggtaagcg ctgctggggg cagccaggag gggacaggca      540
ggagcaatgg gctaggctgt gggtggggaa gatagaactg gagcctgaga aactgcaagc     600
cctttgaaga cagaagccat gagaatcaac atgccaattc ttggcaatcc acttacccac     660
aaccaacatt caccagcatg gttgtactga ttgctaaaat gttaaaatat ttccaaatta     720
agggtgccat gagccccctt tgtgcaccat cctgatgcct gttctagccc ctttaatctc     780
cccattgcct agcagctaga a                                               801

<210>  13
<211>  801
<212>  DNA
<213>  Homo sapiens
<220>
<221>  variation
<222>  (401)..(401)
<223>  /gene="NOTCH4"
      /replace="C"
      /replace="T"
      /db_xref="dbSNP:443198"
<400>  13
tgtctctggg acaagggacc tgccagtgag tgtgccttgc aggagtggga gactggagag      60
aaagggggag ggagagcagg gggggagagg tgaggaagtg agaccaaaga agaaagagag     120
gaagtgaagg agatgaaggg aaacaaatga aggcagagga gggagtgggc aagaatagga     180
agagggacca gtgatgtgag ttttcctctc ctcccctgcc caggtgtgcc cctggcttcc     240
tgggtgagac gtgccagttt cctgacccct gccagaacgc ccagctctgc caaaatggag     300
gcagctgcca agccctgctt cccgctcccc tagggctccc cagctctccc tctccattga     360
cacccagctt cttgtgcact tgcctccctg gcttcactgg ygagagatgc caggccaagc     420
ttgaagaccc ttgtcctccc tccttctgtt ccaaaagggg ccgctgccac atccaggcct     480
cgggccgccc acagtgctcc tgcatgcctg gatggacagg taagcgctgc tgggggcagc     540
caggagggga caggcaggag caatgggcta ggctgtgggt ggggaagata gaactggagc     600
ctgagaaact gcaagccctt tgaagacaga agccatgaga atcaacatgc caattcttgg     660
caatccactt acccacaacc aacattcacc agcatggttg tactgattgc taaaatgtta     720
aaatatttcc aaattaaggg tgccatgagc ccctttgtg caccatcctg atgcctgttc      780
tagccccttt aatctcccca t                                               801

<210>  14
<211>  703
<212>  DNA
<213>  Homo sapiens
<220>
<221>  variation
<222>  (503)..(503)
<223>  /gene="RAB6A"
      /replace="A"
      /replace="G"
      /db_xref="dbSNP:2140893"
<400>  14
ttttattaaa aacaactttt gagccataga tgtgaaatta cttattttaa caaattttct      60
atacaaccac attcattctc tccattttta aatacgactg tacaggtgta gtgtagtttt     120
tttttttctt ttttttaaag acagagtctc actctgttgc ccaggctgga gtgcactggc     180
gtgatctcgg ctcactgcaa cctctgcctc ccaggtttaa gcgattgtcc tgcctcagcc     240
cccccgagta gctggattac aggcgcatgc catcacgcct ggccaatttt gtatttttag     300
tagagacggg gtttcaccat gttggccagg ttagtctcaa actcctgacc tcaggtgatc     360
cgcctgcctt ggcctcccaa agtgctggga ttaccggcgt gagccgctgc tcctggccag     420
ctagtttttt tttttttaaa ttaactttag gagaaacaaa aaatcttgat tcaatttagg     480
tcttttagag aaaggctact agrtttttaa actttagttt tattgactag gtaagttaca     540
ctgaagtagc attagcttgg tttatttgat tttgttttcc tctagaattg catcacctag     600
gtgagacgcc cttggatatg atattgcagt gaggtttgct tgcagctgag cttttctgtt     660
gtgatcccta ggtacttttc tcaactgtct ttgttaccag gat                       703

<210>  15
<211>  401
<212>  DNA
<213>  Homo sapiens
<220>

<221>  variation
<222>  (201)..(201)
<223>  /gene="MPRL"
       /replace="G"
       /replace="T"
       /db_xref="dbSNP:1792174"
<400>  15
gtgtgctaag tactggggta acacagagat ggtaattaat tctttctgga gaaacagcag      60
tgaatgtgac aacctgagtg agggtggaaa gcaggagaat ttctagactc tgaatttcag     120
aagcttccgg aactaaaatg atgtggaagt tgagtgagtc tcggtgggat attgctgctg     180
cacctggtca aggccgttcc ktcagtgttt tcagacgccc tgggaacgcg gctgcagggt     240
ccggtcttcg gtttgcacag ctagaggccg cgcagcagca aaggatgagc ggaaccttgg     300
aaaaggtaac gtagattcca cgcacgcggg gcgcggggag atggcggacg gtgcagagag     360
gggagatggc ggagggtgca gagcggggag gtggcggcgc g                         401

<210>  16
<211>  784
<212>  DNA
<213>  Homo sapiens
<220>
<221>  variation
<222>  (511)..(511)
<223>  /gene="MPRL48"
       /replace="A"
       /replace="T"
       /db_xref="dbSNP:1792160"
<400>  16
ggataattgt aatacaaact gtagtgtacc atttgaagat tcagtgagac aaaagatata      60
aagtgcttgg ccaggtgcct cacgcctgta atcccagcac tttgggaggc cgaggagggc     120
ggatcatgag gtcagatcaa gacaatcctg gctaacccag tgaaacccca tctctactaa     180
aaatacaaaa aattagccgg gcgtggtggc acatgcctgt agtcccagct actcgggagg     240
ctgaggcagg agaattgttt gaaccccgga ggcagaggtt acagtaagcc gagatcatac     300
cactgcactc cagcctgggc gacagagcga gactccgtct caaaaaaaaa aaaaaaaaga     360
taaaatgctc aaaaagtagc agctgtaatt attattatta ttaatactgc tactattcca     420
gaactgcctt gtatgtccat catcaggcta gggcttttct cacactgttt aatccaaaat     480
gaatgtccat tcagtctgat taacatggat wtccatctct gtttcaaccc aattacttgc     540
catttttgct ttgtctatta tgtgagaaat ttcaatggag ttgttggatt ttaatgacgt     600
ttgtcatgtg tctgacaagt atcagagata aaggaatgga agtaaggaag ggaactaaca     660
tttttttactc agtgcctact gtgtgcaagg aagtgtggaa gaggctttac atatgttaat     720
tattttagaa gatggcggca atgtgtaggg aatcaccatt tctactgggc ccaaagccta     780
acat                                                                  784

<210>  17
<211>  401
<212>  DNA
<213>  Homo sapiens
<220>
<221>  variation
<222>  (201)..(201)
<223>  /gene="MPRL48"
       /replace="A"
       /replace="G"
       /db_xref="dbSNP:1792193"
<400>  17
gggatttttt ttttttttta ggattgtgtt gaatctgtag atcaatttgg gaataattga      60
aatcttaatt gtttatattg agtcttctga ttcattaaca caatttctct ctccatttac     120
agtatttaga tattctttat ctggctctgc ttttatgcat tttttggatt tgttggaaaa     180
ctgtaggaga gggaagtaac rtttactccc tacctacctg ctccagtctg ggctctttac     240
atatcttatc tcagtatttc acacagtata ggatgccctc tggttttcct taaataacat     300
taaatcccat gggtagaaat ttcttccctt tgtaattttt tttttttaat tttggagaca      360
gagtcttgct ctgtcaccca ggctggagtg aagtggcgtg a                         401

<210>  18
<211>  101
<212>  DNA
<213>  Homo sapiens
<220>
<221>  variation
<222>  (51)..(51)
<223>  /gene="MPRL48"
       /replace="A"
       /replace="G"
       /db_xref="dbSNP:1051090"
<400>  18
tattgagtgc caaagagaag agcttactgg gtagttagag ttcatcagga racccaaccc      60
ttagatttca taagtaccca ttcccatagc cagtaatgtc c                         101

<210>  19
<211>  121
<212>  DNA
<213>  Homo sapiens
<220>
<221>  variation
<222>  (61)..(61)
<223>  /gene="FLJ11848"
       /replace="G"
       /replace="C"
       /db_xref="dbSNP:3471138"
<400>  19
gcagcaccca ccgccactcc attgatagaa gaaccacaat ctgcaaggac tcccaagcag      60
sctgagcgcc cacaatccca aagtcgtgct gtcccatctc gagaagcaga caccacattc     120
c                                                                     121

<210>  20
<211>  972
<212>  DNA
<213>  Homo sapiens
<220>
<221>  variation
<222>  (497)..(497)
<223>  /gene="FLJ11848"
       /replace="C"
       /replace="T"
       /db_xref="dbSNP:935985"
<400>  20
tatttaacaa gttcttcata ttcaggcacc tccaaacata ttccccttat tcccctccag      60
agaacaggct ttagtactga ggtccttgta aacatttaac aaaactggtt taagaacaaa     120
ctaaaagcat ctatatcctt gccctgtgtt gctggggtga gtttatacaa ggagaagttg     180
gaacaaatgc agtgggcaca atgaccttct tactcagttc aaagtaggtc ttgggatgag     240
agttccacac ggccagtaca gctgactttc tgtgatttcc aaggggtgcc caagacatta     300
aacgccatgg tccttgggaa ggactgatga tgtttctgct cattgttgat cagggtcaaa     360
ccttttcact aaccgggact gctctttcca agaagtcaga ggtcagaaag ctggtagcgt     420
cgtacaagac cgtctcgaca gcatgtgtag atctgcttct cccatgtggc tacctaaaac     480
acaagaattc acagttyaaa ggaagcctgg gattcttcca tttcaaaaag atggaggctt     540
atgattgaca cacactagtc ccatggagtc tgagaaggct ttatgaaggc aacagaatta     600
catccataat tatgtgctta caaaaattgt tggcccatag tgatggatgc atcccaatca     660
ccaatggttg ggatgcacca tgactatata acagagaaac taattggccc aagtggaaag     720
caaacccatt agtattgttc aggtttagca cagatggtct gcctctaggg aggagatggt     780
tcagagatgg gcttgactat tcttgaacct tataaaattg gatgcaaaaa tgttctgaat     840
atgtgtcttt ttttctgagg aggatttcat caactcctca aggaaaacag catgaattct     900
ggagttagtc tcagctttac taccaactag ctgcatgacc tagggcaact ttctcaaccc     960
cccaggcccc ag                                                         972

<210>  21
<211>  401
<212>  DNA
<213>  Homo sapiens
<220>
<221>  variation
<222>  (201)..(201)
<223>  /gene="UCP2"
       /replace="C"
       /replace="T"
       /db_xref="dbSNP:660339"
<400>  21
gttacaggtg aggggatgaa gcctgggagt cttgatggtg tctactctgt tccctcccca      60
aagacacaga cccctcaagg gccagtgttt ggagcatcga gatgactgga ggtgggaagg     120
gcaacatgct tatccctgta gctaccctgt cttggccttg cagatccaag gagaaagtca     180
ggggccagtg cgcgctacag ycagcgccca gtaccgcggt gtgatgggca ccattctgac     240
catggtgcgt actgagggcc cccgaagcct ctacaatggg ctggttgccg gcctgcagcg     300
ccaaatgagc tttgcctctg tccgcatcgg cctgtatgat tctgtcaaac agttctacac     360
caagggctct gagcgtgagt atggagcaag ggtgtaggcc c                         401

<210>  22
<211>  523
<212>  DNA
<213>  Homo sapiens
<220>
<221>  variation
<222>  (201)..(201)
<223>  /gene="UCP3"
       /replace="C"

/replace="T"
/db_xref="dbSNP:2075577"
<400> 22
tatggcccca aaactggggc ctgtggcctt gcagccaggg catccatttc tcccatttcc 60
cattcctccc tccccaccca tttcacatcc ctccccaccg ccttcctaac aggaactttg 120
cccaacatca tgaggaatgc tatcgtcaac tgtgctgagg tggtgaccta cgacatcctc 180
aaggagaagc tgctggacta ycacctgctc actggtgagg ccctgggctc caggcaggca 240
gctctccctc agcagggagg gaacccagaa ctccgtggag tgggcaccac ccagaggcaa 300
ctgggtttta gaaggagcag agagagaagc agtgcctagt aaaaagtgcc aggtacattg 360
tggtgacaag caggaaagac ccctgggctg ggaggctgga gacttgggtt ccagtcccag 420
caccacctct cactatatag ctttaagcag gggtctctca ccttctccga cttcagtttc 480
ctcaaacagc ctgcatgacc tcccacctct gggtcagccc agg 523

<210> 23
<211> 20
<212> DNA
<213> Artificial Sequence
<220>
<223> /gene="TNXB"
/db_xref="dbSNP:2242569"
<400> 23
ggacagggac gggcagcccc 20

<210> 24
<211> 63
<212> DNA
<213> Artificial Sequence
<220>
<223> /gene="TNXB"
/db_xref="dbSNP:2242569"
<400> 24
ctgcccctcg tggaggccgt aatgccggag gtgctccccg tctacggcct ccacgagggg 60
cag 63

<210> 25
<211> 20
<212> DNA
<213> Artificial Sequence
<220>
<223> /gene="TNXB"
/db_xref="dbSNP:2075563"
<400> 25
tccttcaccg tgcagtacaa 20

<210> 26
<211> 63
<212> DNA
<213> Artificial Sequence
<220>
<223> /gene="TNXB"
/db_xref="dbSNP:2075563"
<400> 26
acagatgcat cttgtatttg ccgtttatgt tctacgtaga cagcaaatac aagatgcatc 60
tgt 63

<210> 27
<211> 20
<212> DNA
<213> Artificial Sequence
<220>
<223> /gene="TNXB"
/db_xref="dbSNP:2269428"
<400> 27
ccccaggaaa ggatgaagaa 20

<210> 28
<211>
<212> DNA
<213> Artificial Sequence
<220>
<223> /gene="TNXB"
/db_xref="dbSNP:2269428"
<400> 28
aggctgaggg agtcaggggt cctggggact gagggagtcg gagacccctg actccctcag 60
cct 63

<210>  29
<211>  20
<212>  DNA
<213>  Artificial Sequence
<220>
<223>  /gene="TNXB"
       /db_xref="dbSNP:3749960"
<400>  29
gtgaccccat cctcgtgtcc     20

<210>  30
<211>  63
<212>  DNA
<213>  Artificial Sequence
<220>
<223>  /gene="TNXB"
       /db_xref="dbSNP:3749960"
<400>  30
actccctcag cctctcctgg aaggtcctct ccgactccct catccaggag aggctgaggg     60
agt                                                                   63

<210>  31
<211>  20
<212>  DNA
<213>  Artificial Sequence
<220>
<223>  /gene="TNXB"
       /db_xref="dbSNP:185819"
<400>  31
tgaatctggg gtagggtctg     20

<210>  32
<211>  60
<212>  DNA
<213>  Artificial Sequence
<220>
<223>  /gene="TNXB"
       /db_xref="dbSNP:185819"
<400>  32
tagtgcctca gagtgaccca acccagtgag actccgtgat tgggtcactc tgaggcacta     60

<210>  33
<211>  20
<212>  DNA
<213>  Artificial Sequence
<220>
<223>  /gene="NOTCH4"
       /db_xref="dbSNP:520688"
<400>  33
tcctcccaca gctgatccat     20

<210>  34
<211>  63
<212>  DNA
<213>  Artificial Sequence
<220>
<223>  /gene="NOTCH4"
       /db_xref="dbSNP:520688"
<400>  34
tggctgacac agttgtctgg ggtctgttga cacagtcggt ccagacaact gtgtcagcca     60

<210>  35
<211>  20
<212>  DNA
<213>  Artificial Sequence
<220>
<223>  /gene="NOTCH4"
       /db_xref="dbSNP:2071284"
<400>  35
actccacctt tcacctctgc     20

<210>  36
<211>  21
<212>  DNA
<213>  Artificial Sequence
<220>

<223>  /gene="NOTCH4"
       /db_xref="dbSNP:2071284"
<400>  36
atggatcagc tgtgggagga ggaggagggt gtcgactagg tactcctccc acagctgatc      60
cat                                                                    63

<210>  37
<211>  20
<212>  DNA
<213>  Artificial Sequence
<220>
<223>  /gene="NOTCH4"
       /db_xref="dbSNP:2071283"
<400>  37
caacgagtgc ttccaggacc     20

<210>  38
<211>  63
<212>  DNA
<213>  Artificial Sequence
<220>
<223>  /gene="NOTCH4"
       /db_xref="dbSNP:2071283"
<400>  38
tgcccgcagc tcacaacgtg gggtgcaaca ctcgacgccc gtccacgttg tgagctgcgg      60
gca                                                                    63

<210>  39
<211>  20
<212>  DNA
<213>  Artificial Sequence
<220>
<223>  /gene="NOTCH4"
       /db_xref="dbSNP:2071282"
<400>  39
cgggcttcga gggccatgcc     20

<210>  40
<211>  63
<212>  DNA
<213>  Artificial Sequence
<220>
<223>  /gene="NOTCH4"
       /db_xref="dbSNP:2071282"
<400>  40
acagggcaga ggcactggaa ggaaggtcac ggagacggga cacttccagt gcctctgccc      60
tgt                                                                    63

<210>  41
<211>  20
<212>  DNA
<213>  Artificial Sequence
<220>
<223>  /gene="NOTCH4"
       /db_xref="dbSNP:2071281"
<400>  41
taatggaggg gtgtgtctgg     20

<210>  42
<211>  63
<212>  DNA
<213>  Artificial Sequence
<220>
<223>  /gene="NOTCH4"
       /db_xref="dbSNP:2071281"
<400>  42
ggggcagggt cctgggtcct ggtcctgggt cctgggacgg ggcaggaccc aggaccctgc      60
ccc                                                                    63

<210>  43
<211>  20
<212>  DNA
<213>  Artificial Sequence
<220>
<223>  /gene="NOTCH4"
       /db_xref="dbSNP:415929"

<400> 43
cagccaaccc atgtgttaat 20

<210> 44
<211> 60
<212> DNA
<213> Artificial Sequence
<220>
<223> /gene="NOTCH4"
/db_xref="dbSNP:415929"
<400> 44
ctcgaagccc ggtgggcagt tgacgggtgg cccgaagctc actgcccacc gggcttcgag 60

<210> 45
<211> 20
<212> DNA
<213> Artificial Sequence
<220>
<223> /gene="NOTCH4"
/db_xref="dbSNP:915894"
<400> 45
cacttgcctc cctggcttca 20

<210> 46
<211> 60
<212> DNA
<213> Artificial Sequence
<220>
<223> /gene="NOTCH4"
/db_xref="dbSNP:915894"
<400> 46
ttttggaaca gaaggaggga agggaggaag acaaggtttt tccctccttc tgttccaaaa 60

<210> 47
<211> 20
<212> DNA
<213> Artificial Sequece
<220>
<223> /gene="NOTCH4"
/db_xref="dbSNP:443198"
<400> 47
cacccagctt cttgtgcact 20

<210> 48
<211> 60
<212> DNA
<213> Artificial Sequece
<220>
<223> /gene="NOTCH4"
/db_xref="dbSNP:443198"
<400> 48
gggaggacaa gggtcttcaa aacttctggg aacaggaggg ttgaagaccc ttgtcctccc 60

<210> 49
<211> 20
<212> DNA
<213> Artificial Sequence
<220>
<223> /gene="RAB6A"
/db_xref="dbSNP:2140893"
<400> 49
aaatcttgat tcaatttagg 20

<210> 50
<211> 60
<212> DNA
<213> Artificial Sequence
<220>
<223> /gene="RAB6A"
/db_xref="dbSNP:2140893"
<400> 50
tgtaacttac ctagtcaata ataactgatc cattcaatgt tattgactag gtaagttaca 60

<210> 51
<211> 20
<212> DNA

<213>  Artificial Sequence
<220>
<223>  /gene="MPRL"
       /db_xref="dbSNP:1792174"
<400>  51
tcggtgggat attgctgctg     20

<210>  52
<211>  60
<212>  DNA
<213>  Artificial Sequence
<220>
<223>  /gene="MPRL"
       /db_xref="dbSNP:1792174"
<400>  52
accctgcagc cgcgttccca acccttgcgc cgacgtccca tgggaacgcg gctgcagggt     60

<210>  53
<211>  20
<212>  DNA
<213>  Artificial Sequence
<220>
<223>  /gene="MPRL48"
       /db_xref="dbSNP:1792160"
<400>  53
aatccaaaat gaatgtccat     20

<210>  54
<211>  60
<212>  DNA
<213>  Artificial Sequence
<220>
<223>  /gene="MPRL48"
       /db_xref="dbSNP:1792160"
<400>  54
agcaaaaatg gcaagtaatt ttaatgaacg gtaaaaacga aattacttgc catttttgct     60

<210>  55
<211>  20
<212>  DNA
<213>  Artificial Sequence
<220>
<223>  /gene="MPRL48"
       /db_xref="dbSNP:1792193"
<400>  55
tttttggatt tgttggaaaa     20

<210>  56
<211>  60
<212>  DNA
<213>  Artificial Sequence
<220>
<223>  /gene="MPRL48"
       /db_xref="dbSNP:1792193"
<400>  56
gtaaagagcc cagactggag gaggtcagac ccgagaaatg ctccagtctg ggctctttac     60

<210>  57
<211>  20
<212>  DNA
<213>  Artificial Sequence
<220>
<223>  /gene="MPRL48"
       /db_xref="dbSNP:1051090"
<400>  57
tattgagtgc caaagagaag     20

<210>  58
<211>  63
<212>  DNA
<213>  Artificial Sequence
<220>
<223>  /gene="MPRL48"
       /db_xref="dbSNP:1051090"
<400>  58
ggacattact ggctatggga aaagggtatc ggtcattaca ggttcccata gccagtaatg     60

tcc                                                                 63

<210>  59
<211>  20
<212>  DNA
<213>  Artificial Sequence
<220>
<223>  /gene="FLJ11848"
       /db_xref="dbSNP:3471138"
<400>  59
gcagcaccca ccgccactcc     20

<210>  60
<211>  63
<212>  DNA
<213>  Artificial Sequence
<220>
<223>  /gene="FLJ11848"
       /db_xref="dbSNP:3471138"
<400>  60
ggaatgtggt gtctgcttct cctcttcgtc tgtggtgtaa gggagaagca gacaccacat     60
tcc                                                                 63

<210>  61
<211>  20
<212>  DNA
<213>  Artificial Sequence
<220>
<223>  /gene="FLJ11848"
       /db_xref="dbSNP:935985"
<400>  61
cccatgtggc tacctaaaac     20

<210>  62
<211>  60
<212>  DNA
<213>  Artificial Sequence
<220>
<223>  /gene="FLJ11848"
       /db_xref="dbSNP:935985"
<400>  62
aagcctccat ctttttgaaa aaagtttttc tacctccgaa tttcaaaaag atggaggctt     60

<210>  63
<211>  20
<212>  DNA
<213>  Artificial Sequence
<220>
<223>  /gene="UCP2"
       /db_xref="dbSNP:660339"
<400>  63
cagatccaag gagaaagtca     20

<210>  64
<211>  60
<212>  DNA
<213>  Artificial Sequence
<220>
<223>  /gene="UCP2"
       /db_xref="dbSNP:660339"
<400>  64
gtcagaatgg tgcccatcac cactacccgt ggtaagactg gtgatgggca ccattctgac     60

<210>  65
<211>  20
<212>  DNA
<213>  Artificial Sequence
<220>
<223>  /gene="UCP3"
       /db_xref="dbSNP:2075577"
<400>  65
tggtgaccta cgacatcctc     20

<210>  66
<211>  60
<212>  DNA

<213>  Artificial Sequence
<220>
<223>  /gene="UCP3"
       /db_xref="dbSNP:2075577"
<400>  66
tgcctgcctg gagcccaggg gggacccgag gtccgtccgt ccctgggctc caggcaggca     60